(19) Europäisches Patentamt
European Patent Office
Office européen des brevets

(11) Veröffentlichungsnummer: **0 110 306**
**B1**

(12) # EUROPÄISCHE PATENTSCHRIFT

(45) Veröffentlichungstag der Patentschrift: **04.04.90**

(51) Int. Cl.⁵: **C 07 K 5/08,** C 07 K 5/10, C 07 K 5/02, C 12 Q 1/37

(21) Anmeldenummer: **83111693.4**

(22) Anmeldetag: **23.11.83**

(54) **Chromogene Verbindungen, Verfahren zu ihrer Herstellung und ihre Verwendung.**

(30) Priorität: **27.11.82 DE 3244030**

(43) Veröffentlichungstag der Anmeldung:
**13.06.84 Patentblatt 84/24**

(45) Bekanntmachung des Hinweises auf die Patenterteilung:
**04.04.90 Patentblatt 90/14**

(84) Benannte Vertragsstaaten:
**AT BE CH DE FR GB IT LI LU NL**

(56) Entgegenhaltungen:
**EP-A-0 019 589**
**EP-A-0 025 190**
**EP-A-0 034 122**
**EP-A-0 077 124**
**DE-A-2 322 115**
**DE-A-2 322 116**
**DE-A-2 629 067**
**DE-A-2 724 211**
**DE-A-2 740 323**
**DE-A-3 202 269**
**UNLISTED DRUGS, Band 31, Nr. 6, Juni 1979, Seite 83-b, Nr. S 2266.**

(73) Patentinhaber: **BEHRINGWERKE Aktiengesellschaft**
**Postfach 1140**
**D-3550 Marburg 1 (DE)**

(72) Erfinder: **Heber, Helmut, Dr.**
**Am Ziegenberg 8**
**D-3550 Marburg (DE)**
Erfinder: **Eberle, Reinhard, Dr.**
**Sarnauer Strasse 10**
**D-3551 Lahntal (DE)**
Erfinder: **Teetz, Volker, Dr.**
**An der Tann 20**
**D-6238 Hofheim am Taunus (DE)**

(74) Vertreter: **Klein, Otto, Dr. et al**
**Hoechst AG Zentrale Patentabteilung Postfach 80 03 20**
**D-6230 Frankfurt am Main 80 (DE)**

**EP 0 110 306 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft chromogene Verbindungen der allgemeinen Formel I

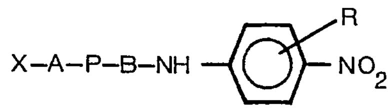

(I)

ihre Säureadditionssalze sowie ein Verfahren zu ihrer Herstellung.

In der Formel bedeuten:

X ein Wasserstoffatom, eine in der Peptidchemie übliche Schutzgruppe oder eine die endständige Aminogruppe irreversibel blockierende Gruppierung;

A und P, gleich oder verschieden, eine aus 2 bis 15 Kohlenstoffatomen mit bis zu 3 Stickstoffatomen, 2 Schwefelatomen und 6 Sauerstoffatomen bestehende alpha-, beta- oder gamma-Aminosäure und P außerdem ein Dipeptid, gebildet aus solchen Aminosäuren;

B Arginin, Homoarginin, Lysin, Homolysin, Ornithin oder Histidin;

R ein Substituent in 2- oder 3-Stellung, der eine Ether-, Ester-, Amid-, Thioester- oder araliphatische Bindung enthält, über die er mit dem aromatischen Kern verbunden sein kann, und aus 1 bis 30 kohlenstoffatomen und der dazugehörigen Anzahl von Wasserstoffatomen besteht, wobei 1 bis 10 Kohlenstoffatome durch Sauerstoff, Stickstoff oder Schwefel und 2 bis 10 Wasserstoffatome durch Gruppen, die Sauerstoff oder Schwefel enthalten, ersetzt sein können sowie ihre Säureadditionssalze.

Die Erfindung betrifft weiterhin die Verwendung dieser Verbindungen als Substrate zum Nachweis und zur quantitativen Bestimmung von hydrolytisch wirkenden Enzymen der Enzymklasse 3.4.21.

Chromogene Verbindungen zur Bestimmung von Proteasen sind aus den DE—OS 32 02 289, 23 22 115, 25 27 932, 25 52 570, 26 29 067, 24 36 543, 26 29 198 und den veröffentlichten EP-Anmeldungen 0 019 589 und 0 034 122 bekannt.

Diese zur Bestimmung von Proteasen verwendeten Substrate nach dem Stand der Technik haben jedoch erheblich Mängel hinsichtlich ihrer Spezifität. So werden beispielsweise zur Bestimmung von Thrombin verwendete chromogene Substrate wie Tos-Gly-Pro-Arg-pNA (Abkürzungen siehe später) und H-D-Phe-Pip-Arg-pNA von anderen Proteasen der Gerinnungs- und Fibrinolysekaskade wie Plasma-Kallikrein, Faktor XII a (M 28.000 aus Placenta), Plasmin und Faktor X a sowie auch Trypsin und Urokonase in erheblichem Umfang gedspalten. Ähnliche Befunde hinsichtlich ihrere Substrat-Enzym-Unspezifität ergeben sich für chromogene Verbindungen, wie sie für andere Proteasen wie Plasma-Kallikrein, Plasmin, Faktor X a und Urokinase im Handel sind. Die Substrate nach DE—OS 24 36 543 mit erhöhter Enzymspezifität haben den Nachteil, daß sie nach Spaltung durch das jeweilige Enzym zur nachträglichen Freisetzung des Chromophors ein Hilfsenzym mit Aminopeptidasewirkung benötigen. Zur Verbesserung dieser Mägel sind in den DE—OS 32 02 289 und 23 22 115 Chromophore beschrieben, bei denen das p-Nitroanilin Substituenten enthält. Diese Substituenten sind Halogenatome, eine Methylgruppe, eine zusätzliche Nitrogruppe, oder es wurde die gesamte Nitroanilingruppe durch eine Aminonaphthyl-, Aminonitronaphthyl-, Aminochinolyl- oder Aminonitrochinolylgruppe ersetzt.

Aufgabe dieser Erfindung ist es, chromogene Verbindungen mit größerer Enzymspezifität zu schaffen, die ohne Hilfsenzyme verwendet werden können.

Gegenstand der Erfindung sind Verbindungen der allgemeinen Formel I und den oben angegebenen Definitionen sowie den folgenden Erläuterungen dazu:

| | |
|---|---|
| Ac | Acetyl |
| Ala | Alanin |
| β-Alanin | beta-Alanin |
| ANBS | 5-Amino-2-Nitrobenzoesäure |
| Arg | Arginin |
| Asn | Asparagin |
| Asp | Asparaginsäure |
| Boc | t-Butyloxycarbonyl |
| But | Aminobuttersäure |
| gamma-But | gamma-Aminobuttersäure |
| Bz | Benzoyl |
| Bzl | Benzyl |
| CHA | Cyclohexylalanin |
| Cys | Cystein |
| DCCI | Dicyclohexylcarbodiimid |
| DCH | Dicyclohexylharnstoff |
| Ddm | 4,4'-Dimethoxybenzhydryl |
| DMF | Dimethylformamid |
| EE | Essigsäureethylester |
| Gln | Glutamin |

| | |
|---|---|
| Glu | Glutaminsäure |
| Gly | Glycin |
| HAc | Essigsäure |
| His | Histidin |
| HOBt | Hydroxybenzotriazol |
| Ile | Isoleucin |
| Leu | Leucin |
| Lys | Lysin |
| Me | Methyl |
| Met | Methionin |
| MM | Molmasse |
| Msc | Methylsulfoethyloxycarbonyl |
| NMM | N-Methylmorpholin |
| Orn | Ornithin |
| PE | Petrolether |
| Pip | Pipecolinsäure |
| pNA | para-Nitroanilin (4-Nitroanilin) |
| Pro | Prolin |
| Pyr | Pyroglutaminsäure |
| Ser | Serin |
| RT | Raumtemperatur |
| tBu | tert. Butyl |
| TCP | 2,4,5-Trichlorphenyl |
| Thr | Threonin |
| TDM | 4,4'-Bis(dimethylamino)-diphenylmethan |
| Tos | Toluolsulfonyl |
| Tyr | Tyrosin |
| Val | Valin |
| Z | Benzyloxycarbonyl |
| ZTE | Benzyloxycarbonyl-amino-2,2,2-Trifluorethyl |

Soweit nicht anders vermerkt, liegen die Aminosäuren in der L-form vor.

Der Ausdruck Aminosäure bedeutet im Rahmen der Erfindung stets eine alpha-, beta- oder gamma-Aminosäure, bestehend aus 2 bis 15 Kohlenstoffatomen mit bis zu 3 Stickstoffatomen, 2 Schwefelatomen und 6 Sauerstoffatomen.

Falls es sich bei A um eine chirale Aminosäure handelt, so liegt sie bevorzugt in der D-form vor, wenn X ein Wasserstoffatom ist.

Falls P chirale Aminosäuren enthält, können sie in der D- oder L-Konfiguration vorliegen. Die unter A und P definierten Aminosäuren sind nicht auf solche beschränkt, wie sie in natürlichen Proteinen oder Peptidantibiotika vorkommen. Auch andere Aminosäuren wie Pipecolinsäure, Cyclohexylalanin, Azetidincarbonsäure, Cysteinsäure, 1-Amino-cyclohexylcarbonsäure, Phenylglycin oder Diphenylglycin kommen in Frage.

R kann eine Ether-, Ester-, Amid-, Thioester- oder araliphatische Bindung enthalten, über die er mit dem aromatischen Kern verbunden sein kann. Der Substituent besteht aus 1 bis 30 Kohlenstoffatomen, wobei 1 bis 10 Kohlenstoffatome durch Sauerstoff, Stickstoff oder Schwefel ersetzt sein können. Ebenso können 2 bis 10 Wasserstoffatome durch Gruppen, die Sauerstoff oder Schwefel enthalten, ersetzt sein.

Die Erfindung betrifft besonders chromogene Verbindungen der allgemeinen Formel II

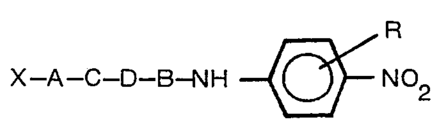

(II)

und ihre Säureadditionssalze.

In der allgemeinen Formel II bedeutet:

X ein Wasserstoffatom, eine in der Peptidchemie übliche Schutzgruppe oder eine die endständige Aminogruppe irreversibel blockierende Gruppierung. In der Peptidchemie übliche Schutzgruppen sind beispielsweise solche nach E. Wünsch in Houben-Weyl, Band XV/1, und The Peptides, Vol. 3, "Protection of Functional Groups in Peptide Synthesis", Academic Press 1981. Vorzugsweise verwendet man Benzyloxycarbonyl, tert. Butyloxycarbonyl, Adamantyloxycarbonyl, Methylsulfonethyloxycarbonyl und 9-Fluorenylmethyloxycarbonyl. Eine irreversibel blockierende Gruppierung kann eine Acyl- oder Sulfonylgruppe, vorzugsweise eine $R_4$-CO-Gruppe, worin $R_4$ einen aliphatischen Kohlenwasserstoffrest mit 1—6 Kohlenstoffatomen, der mit 1—3 Halogenatomen substituiert sein kann, oder eine Alkaryl-Gruppe mit 6—10 Kohlenstoffatomen bedeutet, oder Benzolsulfonyl- oder eine Alkaryl-sulfonyl-Gruppe mit 7—10 Kohlenstoffatomen, besonders jedoch Formyl-, Acetyl-, Benzoyl-, Trifluoracetyl-, Toluolsulfonyl-, Mesyl-, Methoxybenzolsulfonyl-, Succinoyl- oder Maleoyl-, sein;

A eine Aminosäure, deren Seitenkettenfunktion frei oder substituiert sein kann, ausgewählt aus der Gruppe Ala, Cys, Glu, Gln, Gly, His, Ile, Leu, Lys, Phe, Pro, Pyr, Thr, Tyr und Val;

C eine Bindung oder eine Aminosäure, deren Seitenkettenfunktion frei oder substituiert sein kann, ausgewählt aus der Gruppe Ala, Asp, Glu, Gly, Leu, Lys, Ser und Val;

D eine Aminosäure, deren Seitenkettenfunktion frei oder substituiert sein kann, ausgewählt aus der Gruppe Ala, Asp, Glu, Gly, His, Leu, Phe, Pip, Pro, Ser, Thr, Tyr und Val;

B L-Arginin, L-Homoarginin, L-Lysin, L- Homolysin, L-Ornithin oder L-Histindin;

R vorzugsweise $COOR_1$, $CONR_2R_3$, $CONH—(CH_2)_n—N(CH_3)_2$, $CO—Y—OR_1$ und $CO—Y—NR_2R_3$ in 3-Stellung oder $OR_1$ in 2-Stellung des 4-Nitroanilins. Zusätzlich zu R kann noch ein weiterer Substituent vorhanden sein.

$R_1$ ist ein aliphatischer Kohlenwasserstoffrest mit 1 bis 6 Kohlenstoffatomen, ein aromatischer Kohlenwasserstoffrest mit 6 oder 10 kohlenstoffatomen, eine araliphatischer Kohlenwasserstoffrest mit 7 bis 11 Kohlenstoffatomen oder ein alicyclischer Kohlenwasserstoffrest mit 3 bis 8 Kohlenstoffatomen;

$R_2$ ist ein Wasserstoffatom oder ein unter $R_1$ definierter Rest;

$R_3$ ist ein aliphatischer Kohlenwasserstoffrest mit 1 bis 10 Kohlenstoffatomen, ein aromatischer Kohlenwasserstoffrest mit 6 oder 10 Kohlenstoffatomen, ein araliphatischer Kohlenwasserstoffrest mit 7 bis 11 kohlenstoffatomen oder ein alicyclischer Kohlenwasserstoffrest mit 3 bis 8 Kohlenstoffatomen;

Y bedeutet eine alpha-, beta- oder gamma-Aminosäure, deren Seitenkettenfunktion frei oder substituiert sein kann, ausgewählt aus der Gruppe Ala, Asn, Asp, β-Ala, γ-But, Cys, Glu, Gly, Ile, Leu, Lys, Met, Arg, Phe, Pro, Ser, Thr und Tyr; Val;

n bedeutet 1 bis 10.

Als Seitenkettenfunktion der Aminosäuren entsprechend A, C, D und Y kommen Carboxyl-, Hydroxyl-, Mercapto-, Imidazol-, Amino- und Amidgruppen in Betracht. Diese können entsprechend ihrer Reaktivität mit in der Peptidchemie üblichen Schutzgruppen substituiert sein.

Vorzugsweise verwendet man für den Schutz der Säureamidfunktion in Gln und Asn 4,4'-Dimethoxybenzhydryl und 4,4'-Dimethylbenzhydryl. Zum Schutz der Imidazolfunktion in His verwendet man bevorzugt Benzyloxycarbonyl-amino-2,2,2-Trifluorethyl (ZTE), tert. Butylcarbonyl und Tosyl. Zum Schutz der Guanidinofunktion des Arginins kann sie protoniert sein oder durch $N^G$-Tosyl, $N^G$-Nitro, $N^G$-Adamtyloxycarbonyl und $N^G$-Benzyloxycarbonyl substituiert sein. Zum Schutz der ε-Aminofunktion des Lysins verwendet man bevorzugt Benzyloxycarbonyl, tert. Butyloxycarbonyl, Methylsulfoethyloxycarbonyl oder Tosyl. Zum Schutz der Carboxylfunktion in Asp und Glu verwendet man vorzugsweise die Veresterung mit aliphatischen, aromatischen oder araliphatischen Alkoholen wie beispielsweise Methanol, tert. Butanol, Phenol, und Benzylalkohol. Die SH-Funktion in Cystein wird bevorzugt als S-Benzyl oder S-Alkylsulfenyl geschützt. Für die Hydroxyfunktion in Tyrosin, Serin und Threonin kommt als Schutz bevorzugt die Verätherung mit Benzyl oder t-Butyl in Frage.

Die Neuartigkeit der erfindungsgemäßen Substrate liegt in der Derivatisierung der p-Nitroanilidgruppe. Die spektrophotometrischen Eigenschaften der neuen Derivate unterscheiden sich nicht oder nur unwesentlich von denen des 4-Nitroanilins.

Des Nachteil der Unspezifität bei ihrer Verwendung als Enzymsubstrate wiesen die neuen erfindungsgemäßen Verbindungen nicht oder in erheblich geringerem Maße auf als die Substrate des Standes der Technik. Die neuen Substrate zeichnen sich durch eine Derivatisierung der chromogenen Gruppe, in diesem Fall 4-Nitroanilin, aus. Durch Einführung verschiedener Substituenten wird das Chromophor in seiner räumlichen Ausdehnung stark verändert. Dies führt zu einem erheblichen Spezifitätsgewinn bei bestimmten Enzymen, besonders bei Thrombin, Plasmin, Kallikrein, Faktor X a, Urokinase und $C_1$-Esterase.

Gegenstand der Erfindung ist weiterhin ein Verfahren zur Herstellung einer Verbindung der Formel I, dadurch gekennzeichnet, daß ein Peptidderivat der allgemeinen Formel III

$$X—A—P—OH \qquad (III)$$

worin X, A und P die oben angegebene Bedeutung haben und OH eine Hydroxygruppe ist, wobei jedoch X kein Wasserstoffatom bedeutet und zusätzliche funktionelle Gruppen in den Aminosäureseitenketten mit Schutzgruppen substituiert sind, mit einem Aminosäurederivat der allgemeinen Formel IV kondensiert wird,

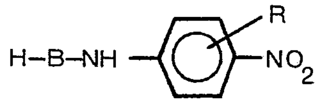

(IV)

worin B die oben genannte Bedeutung besitzt, wobei jedoch zusätzliche funktionelle Gruppen in der Seitenkette der Aminosäure durch Schutzgruppen substituiert sind und R die zu der allgemeinen Formel I gegebene Definition hat. Danach werden die Schutzgruppen gegebenenfalls abgespalten.

Zur Kondensation kommen die in der Peptidchemie üblichen Methoden zur Anwendung, beispielsweise die Azidmethode, die Methode der gemischten Anhydride oder die Methoden der aktivierten Ester (Trichlorphenylester, Pentachlorphenylester, Hydroxysuccinimidester, Nitrophenylester). Bevorzugt verwendet man das Carbodiimidverfahren, gegebenenfalls unter Zusatz von Hydroxybenzotriazol nach Chem. Ber. *103*, 788 (1970).

Die erfindungsgemäßen 5-Amino-2-Nitrobenzoesäurederivate (ANBS-derivate) sind nach bekannten Veresterungs- und Amidbildungsreaktionen zugänglich. Eine zweckmäßige Herstellungsweise für die ANBS-ester ist die Säurechloridmethode mit Thionylchlorid, Phosphortrichlorid oder Phosphorpentachlorid. Bevorzugt verwendet man Thionylchlorid mit dem entsprechenden Alkohol. Die ANBS-ester sind in der Regel in Chloroform oder Essigsäureethylester löslich.

Eine vorteilhafte Herstellungsweise für die ANBS-amide, ANBS-Aminosäureester, ANBS-Aminosäureamide und ANBS-dimethylaminoalkylamide ist die Kondensation nach in der Peptidchemie üblichen Methoden, beispielsweise der Methode der gemischten Anhydride, des Säurechloridverfahrens oder der Carbodiimidmethode. Bevorzugt verwendet man das Carbodiimidverfahren unter Zusatz von Hydroxybenzotriazol. Die Produkte sind nur zum Teil im Essigsäureethylester löslich, können aber oft darin durch Umkristallisation gereinigt werden. Eine Schutzgruppe für die freie Aminogruppe in der ANBS ist nicht notwendig.

Die Umsetzung der ANBS-derivate mit einer der in der allgemeinen Formel I mit B bezeichneten Aminosäuren kann über das Isocyanat des ANBS-derivats erfolgen. Ebenso kann das Aminosäurederivat mittels der Azid-Methode oder der Phosphoroxychloridmethode aktiviert werden. Bevorzugt verwendet man die Phosphoroxychloridmethode. Alle funktionellen Gruppen bis auf die Carboxylgruppe der Aminosäuren sollten mit in der Peptidchemie üblichen Schutzgruppen blockiert sein. Zum Schutz der Guanidinofunktion des Arginins kann sie protoniert sein.

Gegenstand der Erfindung ist weiterhin die Verwendung der erfindungsgemäßen Verbindungen zum Nachweis und zur Bestimmung von Proteinasen. Durch Einwirkung einer Proteinase wird die Bindung zwischen B und dem 4-Nitroanilidderivat gespalten, wodurch ein chromophores Nitroanilinderivat entsteht, daß photometrische gemessen werden kann.

Die Verwendung der Verbindungen der allgemeinen Formel I zum Nachweis und zur Bestimmung von Enzymen stellt im besonderen den Gegenstand der Erfindung dar.

Das Verfahren zur Bestimmung eines hydrolytisch wirksamen Enzyms ist dadurch gekennzeichnet, daß man die Lösung, in der das Enzym bestimmt werden soll, mit einer Verbindung gemäß Formel I versetzt und die Menge oder die Bildungsgeschwindigkeit des abgespaltenen Nitroanilinderivats mißt, die ein direktes Maß für die Aktivität des hydrolytisch wirksamen Enzyms sind.

In der folgenden Tabelle I sind Beispiele erfindungsgemäßer Verbindungen, deren Ausgangsverbindungen gemäß den Formeln III und IV sowie die letzten Reaktionsschritte (Ziffern siehe "Herstellung der neuen Verbindungen" nach Tabelle IX) aufgeführt.

Die Peptidderivate gemäß Formel III wurden nach bekannten Herstellungsverfahren nach E. Wünsch in Houben-Weyl, Band XV/1+2 und The Peptides, Vol. 1, "Major Methods of Peptide Bond Formation", Academic Press 1979 hergestellt.

Die Herstellung der chromogenen Aminosäurederivate der allgemeinen Formel IV wird unten beschrieben.

Alle neuen Verbindungen sind durch Elementaranalyse, Aminosäureanalyse und Dünnschichtchromatographie charakterisiert worden. Die chemische Reinheit wurde durch Dünnschichtchromatographie in verschiedenen Lösungsmittelgemischen festgestellt. Die Racemisierung wurde gaschromatographisch auf einer mit Chirasil-Val$^R$ belegten Glaskapillarsäule nach J. Chromatogr. Sci. *15*, 174 (1977) überprüft und lag immer unter 2 Prozent.

Tabelle I

| | Erfindungsgemäße Verbindung | Ausgangssubstanz gemäß allgem. Formel III und IV | Letzter Reaktionsschritt |
|---|---|---|---|
| 1 | H-D-Val-Leu-Lys-ANBS-methylester x 2HCl | Boc-D-Val-Leu-OH<br>H-Lys(Z)-ANBS-methylester x HCl | 6, 7b |
| 2 | H-D-Phe-Pro-Arg-ANBS-methylester | Boc-D-Phe-Pro-OH<br>H-Arg-ANBS-methylester x 2HCl | 6, 7a |
| 3 | Bz-Ile-Glu(OMe)-Gly-Arg-ANBS-methylester x HCl | Bz-Ile-Glu(OMe)-OH<br>H-Gly-Arg-ANBS-methylester x 2HCl | 6 |
| 4 | Boc-Leu-Ser-Thr-Arg-ANBS-methylester x HCl | Boc-Leu-Ser-Thr-OH<br>H-Arg-ANBS-methylester x 2HCl | 6 |
| 5 | H-D-Leu-Val-Gly-Lys-ANBS-methylester x 2HCl | Boc-D-Leu-Val-Gly-OH<br>H-Lys(Z)-ANBS-methylester x HCl | 6, 7b |
| 6 | H-D-Phe-Pro-Arg-ANBS-ethylester x 2HCl | Boc-D-Phe-Pro-OH<br>H-Arg-ANBS-ethylester x 2HCl | 6, 7a |
| 7 | Tos-Gly-Pro-Arg-ANBS-ethylester x HCl | Tos-Gly-Pro-OH<br>H-Arg-ANBS-ethylester x 2HCl | 6 |
| 8 | Pyr-Gly-Arg-ANBS-ethylester x HCl | Pyr-Gly-OH<br>H-Arg-ANBS-ethylester x 2HCl | 6 |
| 9 | H-D-Phe-Pro-Arg-ANBS-ethylester x 2HCl | Boc-D-Phe-Pro-OH<br>H-Arg-ANBS-ethylester x 2HCl | 6, 7a |
| 10 | H-D-Val-Asp-Arg-ANBS-n-propylester x 2HCl | Boc-D-Val-Asp(OtBu)-OH<br>H-Arg-ANBS-n-propylester x 2HCl | 6, 7a |

Tabelle I (Fortsetzung)

| | Erfindungsgemäße Verbindung | Ausgangssubstanz gemäß allgem. Formel III und IV | Letzter Reaktions-schritt |
|---|---|---|---|
| 11 | H-D-Phe-Tyr-Arg-ANBS-n-propylester x 2HCl | Boc-D-Phe-Tyr-OH<br>H-Arg-ANBS-n-propylester x 2HCl | 6, 7a |
| 12 | H-D-Phe-Pro-Arg-ANBS-isopropylester x 2HCl | Boc-D-Phe-Pro-OH<br>H-Arg-ANBS-isopropylester x 2HCl | 6, 7a |
| 13 | H-D-Cys(sBzl)-Pro-Arg-ANBS-isopropylester x 2HCl | Boc-D-Cys(sBzl)-Pro-OH<br>H-Arg-ANBS-isoproylester x 2HCl | 6, 7a |
| 14 | H-D-Phe-Pro-Arg-ANBS-n-butylester x 2HCl | Boc-D-Phe-Pro-OH<br>H-Arg-ANBS-n-butylester x 2HCl | 6, 7a |
| 15 | Z-D-Leu-Gly-Arg-ANBS-n-butylester x HCl | Z-D-Leu-Gly-OH<br>H-Arg-ANBS-n-butylester x 2HCl | 6 |
| 16 | Pyr-Gly-Arg-ANBS-n-butylester x HCl | Pyr-Gly-OH<br>H-Arg-ANBS-n-butylester x 2HCl | 6 |
| 17 | H-D-Pro-Phe-Arg-ANBS-n-butylester x 2HCl | Boc-D-Pro-Phe-OH<br>H-Arg-ANBS-n-butylester x 2HCl | 6, 7a |
| 18 | H-D-Val-Thr-Arg-ANBS-isobutylester x 2HCl | Boc-D-Val-Thr-OH<br>H-Arg-ANBS-isobutylester x 2HCl | 6, 7a |
| 19 | Msc-Gly-Ser-Lys-ANBS-t-butylester x HCl | Msc-Gly-Ser-OH<br>H-Lys(Z)-ANBS-t-butylester | 6, 7b |
| 20 | H-D-Phe-Tyr-Lys-ANBS n-pentylester x 2HCl | Boc-D-Phe-Tyr-OH<br>H-Lys(Z)-ANBS-n-pentylester | 6, 7b |
| 21 | Bz-Ile-Glu(OMe)-Gly-Arg-ANBS-n-pentylester x HCl | Bz-Ile-Glu(OMe)-OH<br>H-Gly-Arg-ANBS-n-pentylester x 2HCl | 6 |
| 22 | H-D-Phe-Pro-Arg-ANBS-pentyl(2)-ester x 2HCl | Boc-D-Phe-Pro-OH<br>H-Arg-ANBS-pentyl(2)-ester | 6, 7a |

Tabelle I (Fortsetzung)

| | Erfindungsgemäße Verbindung | Ausgangssubstanz gemäß allgem. Formel III und IV | Letzter Reaktionsschritt |
|---|---|---|---|
| 23 | H-D-Lys(Z)-Gly-Arg-ANBS-n-hexylester x 2HCl | Boc-D-Lys(Z)-Gly-OH<br>H-Arg-ANBS-n-hexylester x 2HCl | 6, 7a |
| 24 | N-D-Phe-Pro-Arg-ANBS-benzylester x 2HCl | Boc-D-Phe-Pro-OH<br>H-Arg-ANBS-benzylester x 2HCl | 6, 7a |
| 25 | Tos-Gly-Pro-Arg-ANBS-benzylester x HCl | Tos-Gly-Pro-OH<br>H-Arg-ANBS-benzylester x 2HCl | 6 |
| 26 | Z-D-Leu-Gly-Arg-ANBS-benzylester x HCl | Z-D-Leu-Gly-OH<br>H-Arg-ANBS-benzylester x 2HCl | 6 |
| 27 | H-D-Phe-Pro-Arg-ANBS-methylamid x 2HCl | Boc-D-Phe-Pro-OH<br>H-Arg-ANBS-methylamid x 2HCl | 6, 7a |
| 28 | H-D-Val-Leu-Lys-ANBS-methyamid x 2HCl | Boc-D-Val-Leu-OH<br>H-Lys(Z)-ANBS-methylamid x HCl | 6, 7b |
| 29 | Tos-Gly-Pro-Arg-ANBS-methylamid x HCl | Tos-Gly-Pro-OH<br>H-Arg-ANBS-methylamid x 2HCl | 6 |
| 30 | Z-D-Leu-Gly-Arg-ANBS-methylamid x HCl | Z-D-Leu-Gly-OH<br>H-Arg-ANBS-methylamid x 2HCl | 6 |
| 31 | Bz-Ile-Glu(OMe)-Gly-Arg-ANBS-methylamid x HCl | Bz-Ile-Glu(OMe)-OH<br>H-Gly-Arg-ANBS-methylamid x 2HCl | 6 |
| 32 | Pyr-Gly-Arg-ANBS-methylamid x HCl | Pyr-Gly-OH<br>H-Arg-ANBS-methylamid x 2HCl | 6 |
| 33 | H-D-Pro-Phe-Arg-ANBS-methylamid x 2HCl | Boc-[illegible]-Pro-Phe-OH<br>H-Arg-ANBS-methylamid x 2HCl | 6, 7a |
| 34 | H-D-Ala-Gly-Arg-ANBS-methylamid x 2HCl | Boc-D-Ala-Gly-OH<br>H-Arg-ANBS-methylamid x 2HCl | 6, 7a |
| 35 | H-D-Phe-Tyr-Arg-ANBS | Boc-D-Phe-Tyr-OH | 6, 7a |

Tabelle I (Fortsetzung)

| | Erfindungsgemäße Verbindung | Ausgangssubstanz gemäß allgem. Formel III und IV | Letzter Reaktionsschritt |
|---|---|---|---|
| | ethylamid x 2HCl | H-Arg-ANBS-ethylamid x 2HCl | |
| 36 | Boc-His-(Boc)-Gly-Arg-ANBS-ethylamid x HCl | Boc-His-(Boc)-Gly-OH<br>H-Arg-ANBS-ethylamid x 2HCl | 6 |
| 37 | H-D-Phe-Ser(OBzl)-Arg-ANBS-n-propylamid x 2HCl | Boc-D-Phe-Ser(OBzl)-OH<br>H-Arg-ANBS-n-propylamid x 2HCl | 6, 7a |
| 38 | H-D-Phe-Pro-Arg-ANBS-isopropylamid x 2HCl | Boc-D-Phe-Pro-OH<br>H-Arg-ANBS-isopropylamid x 2HCl | 6, 7a |
| 39 | H-D-Val-Tyr(OBzl)-Arg-ANBS-isopropylamid x 2HCl | Boc-D-Val-Tyr(OBzl)-OH<br>H-Arg-ANBS-isopropylamid x 2HCl | 6, 7a |
| 40 | Tos-Gly-Pro-Arg-ANBS-isopropylamid x HCl | Tos-Gly-Pro-OH<br>H-Arg-ANBS-isopropylamid x 2HCl | 6 |
| 41 | Z-D-Leu-Gly-Arg-ANBS-isopropylamid x HCl | Z-D-Leu-Gly-OH<br>H-Arg-ANBS-isopropylamid x 2HCl | 6 |
| 42 | Bz-Ile-Glu(OMe)-Gly-Arg-ANBS-isopropylamid x HCl | Bz-Ile-Glu(OMe)-OH<br>H-Gly-Arg-ANBS-isopropylamid x 2HCl | 6 |
| 43 | Pyr-Gly-Arg-ANBS-isopropylamid x HCl | Pyr-Gly-OH<br>H-Arg-ANBS-isopropylamid x 2HCl | 6 |
| 44 | H-D-Pro-Phe-Arg-ANBS-isopropylamid x 2HCl | Boc-D-Pro-Phe-OH<br>H-Arg-ANBS-isopropylamid x 2HCl | 6, 7a |
| 45 | H-D-Phe-Pip-Arg-ANBS-isoproylamid x 2HCl | Boc-D-Phe-Pip-OH<br>H-Arg-ANBS-isopropylamid x 2HCl | 6, 7a |
| 46 | H-D-Glu-Gly-Leu-Arg-ANBS-isopropylamid x 2HCl | Boc-D-Glu(OBzl)-Gly-Leu-OH<br>H-Arg-ANBS-isopropylamid x 2HCl | 6, 7c |
| 47 | H-D-Glu-Gly-Leu-Lys-ANBS-isopropylamid x 2HCl | Boc-D-Glu(OBzl)-Gly-Leu-OH<br>H-Lys(Z)-ANBS-isopropylamid x HCl | 6, 7c |

Tabelle I (Fortsetzung)

| | Erfindungsgemäße Verbindung | Ausgangssubstanz gemäß allgem. Formel III und IV | Letzter Reaktionsschritt |
|---|---|---|---|
| 48 | Bz-Ile-Asp-Ala-Arg-ANBS-n-butylamid x HCl | Bz-Ile-Asp(OtBu)-OH<br>H-Ala-Arg-ANBS-n-butylamid x 2HCl | 6, 7a |
| 49 | H-D-Phe-Tyr-Arg-ANBS t-butylamid x 2HCl | Boc-D-Phe-Tyr-OH<br>H-Arg-ANBS-t-butylamid x 2HCl | 6, 7a |
| 50 | H-D-Leu-Val-Gly-Lys-ANBS-isobutylamid x 2HCl | Boc-D-Leu-Val-Gly-OH<br>H-Lys(Z)-ANBS-isobutylamid x HCl | 6, 7b |
| 51 | H-D-Phe-Ser(OBzl)-Orn-ANBS-n-pentylamidx2HCl | Boc-D-Phe-Ser(OBzl)-OH<br>H-Orn(Z)-ANBS-n-pentylamid x HCl | 6, 7b |
| 52 | H-D-Phe-Pro-His-ANBS-neopentylamid x 2 HCl | Boc-D-Phe-Pro-OH<br>H-His(ZTE)-ANBS-neopentylamid x HCl | 6, 7b |
| 53 | H-D-Phe-Pro-Arg-ANBS-neopentylamid x 2HCl | Boc-D-Phe-Pro-OH<br>H-Arg-ANBS-neopentylamid x 2HCl | 6, 7a |
| 54 | H-D-Val-Leu-Lys-ANBS-neopentylamid x 2HCl | Boc-D-Val-Leu-OH<br>H-Lys(Z)-ANBS-neopentylamid x HCl | 6, 7a |
| 55 | Z-D-Val-Leu-Arg-ANBS-neopentylamid x HCl | Z-D-Val-Leu-OH<br>H-Arg-ANBS-neopentylamid x 2HCl | 6 |
| 56 | H-D-Phe-His(ZTE)-Arg-ANBS-neopentylamid x 2HCl | Boc-D-Phe-His(ZTE)-OH<br>H-Arg-ANBS-neopentylamid x 2HCl | 6, 7a |
| 57 | H-D-Pro-Phe-Arg- ANBS-neopentylamid x 2HCl | Boc-D-Pro-Phe-OH<br>H-Arg-ANBS-neopentylamid x 2HCl | 6, 7a |
| 58 | Tos-Val-Pro-Arg-ANBS-n-hexylamid x HCl | Tos-Val-Pro-OH<br>H-Arg-ANBS-n-hexylamid x 2HCl | 6 |
| 59 | Tos-Gly-Val-Orn-ANBS-n-octylamid x HCl | Tos-Gly-Val-OH<br>H-Orn(Z)-ANBS-n-octylamid x HCl | 6, 7b |
| 60 | H-D-Phe-Pro-Arg-ANBS- | Boc-D-Phe-Pro-OH | 6, 7a |

Tabelle I (Fortsetzung)

| | Erfindungsgemäße Verbindung | Ausgangssubstanz gemäß allgem. Formel III und IV | Letzter Reaktionsschritt |
|---|---|---|---|
| | n-decylamid x 2 HCl | H-Arg-ANBS-n-decylamid x 2HCl | |
| 61 | H-D-Phe-Pro-Arg-ANBS-benzylamid x 2HCl | Boc-D-Phe-Pro-OH<br>H-Arg-ANBS-benzylamid x 2HCl | 6, 7a |
| 62 | Tos-Gly-Pro-Arg-ANBS-benzylamid x HCl | Tos-Gly-Pro-OH<br>H-Arg-ANBS-benzylamid x 2HCl | 6 |
| 63 | Z-D-Leu-Gly-Arg-ANBS benzylamid x HCl | Z-D-Leu-Gly-OH<br>H-Arg-ANBS-benzylamid x 2HCl | 6 |
| 64 | Bz-Ile-Glu(OMe)-Gly-Arg-ANBS-benzylamid x HCl | Bz-Ile-Glu(OMe)-OH<br>H-Gly-Arg-ANBS-benzylamid x 2HCl | 6 |
| 65 | Pyr-Gly-Arg-ANBS-benzylamid x HCl | Pyr-Gly-OH<br>H-Arg-ANBS-benzylamid x 2HCl | 6 |
| 66 | H-D-Pro-Phe-Arg-ANBS-benzylamid x 2HCl | Boc-D-Phe-Pro-OH<br>H-Arg-ANBS-benzylamid x 2HCl | 6, 7a |
| 67 | Bz-Ile-Asp-Gly-Arg-ANBS-benzylamid x HCl | Bz-Ile-Asp(OtBu)-OH<br>H-Gly-Arg-ANBS-benzylamid x 2HCl | 6, 7a |
| 68 | H-Gly-Val-Orn-ANBS-benzylamid x 2HCl | Z-Gly-Val-OH<br>H-Orn(Z)-ANBS-benzylamid x HCl | 6, 7b |
| 69 | Ac-Phe-Glu(OtBu)-Arg-ANBS-phenethylamid x HCl | Ac-Phe-Glu(OtBu)-OH<br>H-Arg-ANBS-phenethylamid x 2HCl | 6 |
| 70 | H-D-Phe-Pro-Arg-ANBS-phenethylamid x 2HCl | Boc-D-Phe-Pro-OH<br>H-Arg-ANBS-phenethylamid x 2HCl | 6, 7a |
| 71 | H-D-Phe-Pro-Arg-ANBS-phenethylamid x 2HCl | Boc-D-Phe-Pro-OH<br>H-Arg-ANBS-phenethylamid x 2HCl | 6, 7a |
| 72 | Tos-Gly-Pro-Arg-ANBS-cyclopentylamid x HCl | Tos-Gly-Pro-OH<br>H-Arg-ANBS-cyclopentylamid x 2HCl | 6 |

Tabelle I (Fortsetzung)

| | Erfindungsgemäße Verbindung | Ausgangssubstanz gemäß allgem. Formel III und IV | Letzter Reaktionsschritt |
|---|---|---|---|
| 73 | H-D-Phe-Pro-Arg-ANBS-cyclohexylamid x 2HCl | Boc-D-Phe-Pro-OH<br>H-Arg-ANBS-cyclohexylamid x 2HCl | 6, 7a |
| 74 | Ac-Phe-Glu(OtBu)-Arg-ANBS-cyclohexylamid x HCl | Ac-Phe-Glu(OtBu)-OH<br>H-Arg-ANBS-cyclohexylamid x 2HCl | 6 |
| 75 | H-D-Phe-Pro-Arg-ANBS-diethylamid x 2HCl | Boc-D-Phe-Pro-OH<br>H-Arg-ANBS-diethylamid x 2HCl | 6, 7a |
| 76 | Tos-Gly-Pro-Arg-ANBS-diisopropylamid x HCl | Tos-Gly-Pro-OH<br>H-Arg-ANBS-diisopropylamid x 2HCl | 6 |
| 77 | H-D-Phe-Pro-Arg-ANBS-diisopropylamid x 2HCl | Boc-D-Phe-Pro-OH<br>H-Arg-ANBS-diisopropylamid x 2HCl | 6, 7a |
| 78 | H-D-Phe-Pro-Arg-ANBS-dicyclohexylamid x 2HCl | Boc-D-Phe-Pro-OH<br>H-Arg-ANBS-dicyclohexylamid x 2HCl | 6, 7a |
| 79 | H-D-Phe-His(ZTE)-Arg-ANBS-dimethylaminoethylamid x 3HCl | Boc-D-Phe-His(ZTE)-OH<br>H-Arg-ANBS-dimethylaminoethylamid x 3HCl | 6, 7a |
| 80 | H-D-Phe-Pro-Arg-ANBS-dimethylaminopropylamidx3HCl | Boc-D-Phe-Pro-OH + H-Arg-ANBS-dimethylaminopropylamid x 3HCl | 6, 7a |
| 81 | Bz-Ile-Glu(OMe)-Gly-Arg-ANBS-diemthylaminopropylamid x 2HCl | Bz-Ile-Glu(OMe)-OH<br>H-Gly-Arg-ANBS-dimethylaminopropylamid x 3HCl | 6 |
| 82 | H-D-Phe-Pro-Arg-2-methoxy-pNA x 2HCl | Boc-D-Phe-Pro-OH<br>H-Arg-2-methoxy-pNA x 2HCl | 6, 7a |
| 83 | Ac-Phe-Glu(OtBu)-Arg-2-methoxy-pNA x HCl | Ac-Phe-Glu(OtBu)-OH<br>H-Arg-2-methoxy-pNA x 2HCl | 6 |
| 84 | Z-D-Leu-Gly-Arg-2-methoxy- | Z-D-Leu-Gly-OH | 6 |

Tabelle I (Fortsetzung)

| | Erfindungsgemäße Verbindung | Ausgangssubstanz gemäß allgem. Formel III und IV | Letzter Reaktionsschritt |
|---|---|---|---|
| | pNA x HCl | H-Arg-2-methoxy-pNA x 2HCl | |
| 85 | Tos-Gly-Pro-Arg-ANBS- | Tos-Gly-Pro-OH | 6 |
| | 2-methoxy-pNA x HCl | H-Arg-2-methoxy-pNA x 2HCl | |
| 86 | Bz-Ile-Glu(OMe)-Gly-Arg- | Bz-Ile-Glu(OMe)-OH | 6 |
| | 2-methoxy-pNA xHCl | H-Gly-Arg-2-methoxy-pNA x 2HCl | |
| 87 | Z-D-Val-Leu-Arg-2- | Z-D-Val-Leu-OH | 6 |
| | methoxy-pNA x HCl | H-Arg-2-methoxy-pNA x 2HCl | |
| 88 | H-D-Val-Leu-Arg-2- | Z-D-Val-Leu-OH | 6, 7b |
| | methoxy-pNA x HCl | H-Arg-2-methoxy-pNA x 2HCl | |
| 89 | Pyr-Gly-Arg-2- | Pyr-Gly-OH | 6 |
| | methoxy-pNA x CHl | H-Arg-2-methoxy-pNA x 2HCl | |
| 90 | H-D-Pro-Phe-Arg-2- | Boc-D-Pro-Phe-OH | 6, 7a |
| | methoxy-pNA x 2HCl | H-Arg-2-methoxy-pNA- 2HCl | |
| 91 | Boc-D-Lys(Z)-Gly-Arg- | Boc-D-Lys(Z)-Gly-OH | 6 |
| | 2-methoxy-pNA x HCl | H-Arg-2-methoxy-pNA x 2HCl | |
| 92 | H-D-Lys(Z)-Gly-Arg- | Boc-D-Lys(Z)-Gly-OH | 6, 7a |
| | 2-methoxy-pNA x 2HCl | H-Arg-2-methoxy-pNA x 2HCl | |
| 93 | Boc-D-Lys(Z)-Gly-Gly-Arg- | Boc-D-Lys(Z)-Gly-OH | 6 |
| | 2-methoxy-pNA x HCl | H-Gly-Arg-2-methoxy-pNA x 2HCl | |
| 94 | Bz-Ile-Asp-Ala-Arg-2- | Bz-Ile-Asp(OtBu)-OH | 6, 7a |
| | methoxy-pNA x HCl | H-Arg-2-methoxy-pNA x 2HCl | |
| 95 | H-D-Thr-Ala-Thr-Arg- | Boc-D-Thr-Ala-Thr-OH | 6, 7a |
| | 2-methoxy-pNA x 2HCl | H-Arg-2-methoxy-pNA x 2HCl | |
| 96 | Boc-Leu-Ser-Thr-Arg- | Boc-Leu-Ser-Thr-OH | 6 |
| | 2-methoxy-pNA x HCl | H-Arg-2-methoxy-pNA x 2HCl | |

Tabelle I (Fortsetzung)

| | Erfindungsgemäße Verbindung | Ausgangssubstanz gemäß allgem. Formel III und IV | Letzter Reaktionsschritt |
|---|---|---|---|
| 97 | H-D-Phe-Pro-Arg-2-butoxy-pNA x 2HCl | Boc-D-Phe-Pro-OH<br>H-Arg-2-butoxy-pNA x 2HCl | 6, 7a |
| 98 | Pyr-Gly-Arg-2-butoxy-pNA x HCl | Pyr-Gly-OH<br>H-Arg-2-butoxy-pNA x 2HCl | 6 |
| 99 | Z-D-Leu-Gly-Arg-2-butoxy-pNA x HCl | Z-D-Leu-Gly-OH<br>H-Arg-2-butoxy-pNA x 2HCl | 6 |
| 100 | H-D-Val-Leu-Arg-2-butoxy-pNA x 2HCl | Z-D-Val-Leu-OH<br>H-Arg-2-butoxy-pNAx 2HCl | 6, 7b |
| 101 | H-D-Lys(Z)-Gly-Arg-2-butoxy-pNA x 2HCl | Boc-D-Lys(Z)-Gly-OH<br>H-Arg-2-butoxy-pNA x 2HCl | 6, 7a |
| 102 | Tos-Gly-Pro-Arg-ANBS-Gly-methylester x HCl | Tos-Gly-Pro-OH<br>H-Arg-ANBS-Gly-methylester x 2HCl | 6 |
| 103 | H-D-Phe-Pro-Arg-ANBS-Gly-ethylester x 2HCl | Boc-D-Phe-Pro-OH<br>H-Arg-ANBS-Gly-methylester x 2HCl | 6, 7a |
| 104 | Pyr-Gly-Arg-ANBS-Ala-methylester x HCl | Pyr-Gly-OH<br>H-Arg-ANBS-Ala-methylester x 2HCl | 6 |
| 105 | Boc-Leu-Ser-Thr-Arg-ANBS-Ala-methylester x HCl | Boc-Leu-Ser-Thr-OH<br>H-Arg-ANBS-Ala-methylester x 2HCl | 6 |
| 106 | H-D-Lys(Z)-Gly-Arg-ANBS-β-Ala-ethylester x 2HCl | Boc-D-Lys(Z)-Gly-OH<br>H-Arg-ANBS-β-Ala-ethylester x 2HCl | 6, 7a |
| 107 | Tos-Gly-Pro-Arg-ANBS-Ala-ethylester x HCl | Tos-Gly-Pro-OH<br>H-Arg-ANBS-Ala-ethylester x 2HCl | 6 |
| 108 | H-D-Phe-Pro-Arg-ANBS-γ-But-ethylester x 2HCl | Boc-D-Phe-Pro-OH<br>H-Arg-ANBS-γ-But-ethylester x 2HCl | 6, 7a |
| 109 | H-D-Tyr-His(ZTE)-Arg- | Boc-D-Tyr-His(ZTE)-OH | 6, 7a |

Tabelle I (Fortsetzung)

| | Erfindungsgemäße Verbindung | Ausgangssubstanz gemäß allgem. Formel III und IV | Letzter Reaktions-schritt |
|---|---|---|---|
| | ANBS-γ-But-ethylester x 2HCl | H-Arg-ANBS-γ-But-ethylester x 2HCl | |
| 110 | H-D-Phe-Pro-Arg-ANBS-Val-methylester x 2HCl | Boc-Arg-ANBS-γ-But-ethylester x 2HCl<br>H-Arg-ANBS-Val-methylester x 2HCl | 6, 7a |
| 111 | Pyr-Gly-Arg-ANBS-Val-methylester x HCl | Pyr-Gly-OH<br>H-Arg-ANBS-Val-methylester x 2HCl | 6 |
| 112 | Z-D-Leu-Gly-Arg-ANBS-Leu-methlyester x HCl | Z-D-Leu-Gly-OH<br>H-Arg-ANBS-Leu-methylester x 2HCl | 6 |
| 113 | H-D-Thr-Ala-Thr-Arg-ANBS-Ile-methylester x 2HCl | Boc-D-Thr-Ala-Thr-OH<br>H-Arg-ANBS-Ile-methylester x 2HCl | 6, 7a |
| 114 | H-D-Val-Lys-Val-Arg-ANBS-Ile-methylester x 3HCl | Z-D-Val-Lys(Z)-Val-OH<br>H-Arg-ANBS-Ile-methylester x 2HCl | 6, 7b |
| 115 | H-D-Phe-Pro-Arg-ANBS-Ile-methylester x 2HCl | Boc-D-Phe-Pro-OH<br>H-Arg-ANBS-Ile-methylester x 2HCl | 6, 7a |
| 116 | H-D-Phe-Tyr-Arg-ANBS-Pro-methylester x 2HCl | Boc-D-Phe-Tyr-OH<br>H-Arg-ANBS-Pro-methylester x 2HCl | 6, 7a |
| 117 | Ac-Gln(Ddm)-Leu-Gly-Arg-ANBS-Phe-methylester x HCl | Hc-Gln(Ddm)-Leu-Gly-OH<br>H-Arg-ANBS-Phe-methylester x 2HCl | 6 |
| 118 | H-D-Phe-Pro-Arg-ANBS-Lys(Z)-methylester x 2HCl | Boc-D-Phe-Pro-OH<br>H-Arg-ANBS-Lys(Z)-methylester x 2HCl | 6, 7a |
| 119 | Boc-D-Lys(Z)-Gly-Arg-ANBS-Lys(Z)-methylester x HCl | Boc-D-Lys(Z)-Gly-OH<br>H-Arg-ANBS-Lys(Z)-methylester x 2HCl | 6 |
| 120 | H-D-Lys(Z)-Gly-Arg-ANBS-Lys(Z)-methylester x 2HCl | Boc-D-Lys(Z)-Gly-OH<br>H-Arg-ANBS-Lys(Z)-methylester x 2HCl | 6, 7a |
| 121 | H-D-Phe-Pro-Arg-ANBS-Ser-methylester x 2HCl | Boc-D-Phe-Pro-OH<br>H-Arg-ANBS-Ser-methylester x 2HCl | 6, 7a |

Tabelle I (Fortsetzung)

| | Erfindungsgemäße Verbindung | Ausgangssubstanz gemäß allgem. Formel III und IV | Letzter Reaktionsschritt |
|---|---|---|---|
| 122 | H-D-Ala-Gly-Arg-ANBS-Thr-methylester x 2HCl | Boc-D-Ala-Gly-OH<br>H-Arg-ANBS-Thr-methylester x 2HCl | 6, 7a |
| 123 | H-D-Tyr-His(ZTE)-Arg-ANBS-Tyr-methylester x 2HCl | Boc-D-Tyr-His(ZTE)-OH<br>H-Arg-ANBS-Tyr-methylester x 2HCl | 6, 7a |
| 124 | H-D-Phe-Pro-Arg-ANBS-Arg-methylester x 3HCl | Boc-D-Phe-Pro-OH<br>H-Arg-ANBS-Arg($NO_2$)-methylester x 2HCl | 6, 7c |
| 125 | H-D-Phe-Pro-Arg-ANBS-Glu-methylester x 2HCl | Boc-D-Phe-Pro-OH<br>H-Arg-ANBS-Glu-methylester x 2HCl | 6, 7a |
| 126 | H-D-Val-Leu-Lys-ANBS-Asp-methylester x 2HCl | Z-D-Val-Leu-OH<br>H-Lys(Z)-ANBS-Asp-methylester x HCl | 6, 7b |
| 127 | Pyr-Gly-Arg-ANBS-Cys-methylester x HCl | Pyr-Gly-OH<br>H-Arg-ANBS-Cys-methylester x 2HCl | 6 |
| 128 | H-D-Pro-Phe-Arg-ANBS-Met-methylester x 2HCl | Boc-D-Pro-Phe-OH<br>H-Arg-ANBS-Met-methylester x 2HCl | 6, 7a |
| 129 | H-D-Phe-Pro-Arg-ANBS-Ala-isopropylamid x 2HCl | Boc-D-Phe-Pro-OH<br>H-Arg-ANBS-Ala-isopropylamid x 2HCl | 6, 7a |
| 130 | Z-D-Leu-Gly-Arg-ANBS-Ile-methylamid x HCl | Z-D-Leu-Gly-OH<br>H-Arg-ANBS-Ile-methylamid x 2HCl | 6 |
| 131 | H-D-Val-Tyr(OBzl)-Arg-ANBS-Ile-diisopropylamid x 2HCl | Boc-D-Val-Tyr(OBzl)-OH<br>H-Arg-ANBS-Ile-diisopropylamid x 2HCl | 6, 7a |
| 132 | H-Gly-Val-Arg-ANBS-Ile-dicyclohexylamid x 2HCl | Z-Gly-Val-OH<br>H-Arg-ANBS-Ile-dicyclohexylamid x 2HCl | 6, 7b |
| 133 | H-D-Phe-Pro-Arg-ANBS-Arg-isopropylamid x 3HCl | Boc-D-Phe-Pro-OH<br>H-Arg-ANBS-Arg($NO_2$)-isopropylamid x 2HCl | 6, 7c |
| 134 | H-D-Phe-Pip-Arg-ANBS-Arg-isopropylamid x 3HCl | Boc-D-Phe-Pip-OH<br>H-Arg-ANBS-Arg($NO_2$)-isopropylamid x 2HCl | 6, 7c |

Die folgenden Tabellen II bis IX zeigen Ergebnisse von enzymatischen Messungen. Diese zeigen besonders den Vorteil der neuen Verbindungen gegenüber denen des Standes der Technik.

Die Konzentrationsangaben für die Enzyme beziehen sich auf die Stammlösungen, die eingesetzt wurden. Diese Stammlösungen werden in Test im Verhältnis 1:10 mit Puffer verdünnt. Der pH-Wert des Puffers liegt beim Aktivitätsoptimum des entsprechenden Enzyms.

Alle eingesetzten Enzyme mit Ausnahme von Trypsin sind Präparate der Behringwerke AG. Trypsin stammt von SERVA. Als Aktivator bezeichnen wir den 1:1-Komplex zwischen Human-Plasminogen und Streptokinase.

Tabelle II

Spezifitätsänderung der Enzymsubstrate in Abhängigkeit von der Größe des Substituenten R der allgemeinen Formel I am Beispiel der H-D-Phe-Pro-Arg-ANBS-ester in $\Delta$ OD/min bei 405 nm.

| Synthetische Substrate x 2 HCl 3,0 mMol/l | Thrombin 6 IU/ml | Plasmin 2CTA/ml | Kallikrein 0,85BAEE/ml | Urokinase 5000IU/ml | Trypsin 1,4γ/ml |
|---|---|---|---|---|---|
| H-D-Phe-Pro-Arg-ANBS- | | | | | |
| -methylester | 0,335 | 0,200 | 0,230 | 0,090 | 0,160 |
| -ethylester | 0,360 | 0,180 | 0,180 | 0,070 | 0,110 |
| -isopropylester | 0,355 | 0,130 | 0,110 | 0,050 | 0,090 |
| -n-butylester | 0,365 | 0,100 | 0,095 | 0,050 | 0,080 |
| -pentyl(2)ester | 0,345 | 0,080 | 0,060 | 0,030 | 0,050 |
| -benzylester | 0,350 | 0,025 | 0,035 | 0,015 | 0,035 |

Während die Sensibilität der verschiedenen Peptid-ANBS-ester gegenüber Thrombin gleich bleibt und in einer für enzymatische Messungen brauchbaren Größenordnung liegt, steigt die Spezifität der einzelnen Enzymsubstrate mit der Größe des Alkoholrestes deutlich an.

Tabelle III

Vergleich der Spaltungsgeschwindigkeiten verschiedener Thrombin-Substrate nach Kontaktphasenaktivierung von menschlichem Citratplasma in $\Delta$ OD/min bei 405 nm. Die Spaltung durch Thrombin ist im Vergleich gegenübergestellt.

| Synthetische Substrate x 2 HCL 3 mMol/1 | Plasma nach Kontaktphasenaktivierung 10 µl | Thrombin 6 IU/ml |
|---|---|---|
| Substrate für Thrombin nach dem Stand der Technik: | | |
| H-D-CHA-But-Arg-pNA | 0,325 | 0,300 |
| H-D-Phe-Pro-Arg-pNA | 0,100 | 0,350 |
| H-D-Phe-Pip-Arg-pNA | 0,090 | 0,350 |
| Erfindungsgem. Substr.für Thrombin: | | |
| H-D-Phe-Pro-Arg-ANBS-isopropylamid | 0,037 | 0,350 |
| H-D-Phe-Pro-Arg-ANBS-γ-But-ethylester | 0,028 | 0,360 |

Aktiviert man menschliches Citratplasma in Abwesenheit von Phospholipid und Calcium mit Dextransulfat, so werden nach dem Stand der Erkenntnis nur die Proteasen Faktor XII, Faktor XI sowie Plasma-Kallikrein aktiviert. Vergleicht man in diesem Testsystem im Handel befindliche Thrombinsubstrate mit den erfindungsgemäßen Substraten für Thrombin, so zeigt sich daß die Substrate nach dem Stand der Technik um ein vielfaches schneller gespalten werden als die neuen Substrate.

Die Tabellen IV bis IX zeigen einen Vergleich der Spaltungsgeschwindigkeiten durch verschiedene proteolytische Enzyme von Substraten nach dem Stand der Tecknik und einigen erfindungsgemäßen Verbindungen. Aus diesen Tabellen geht die Überlegenheit der erfindungsgemäßen Enzymsubstrate hervor. So ist die Sensibilität der einzelnen Substrate gegenüber einem bestimmten Enzym ausreichend und in der gleichen Größenordnung wie bei den Substraten des Standes der Technik. Die Unempfindlichkeit der erfindungsgemäßen Verbindungen gegenüber anderen Proteasen, die ebenfalls im Plasma vorkommen können, steigt je nach Art und Größe des Substituenten in den Peptid-ANBS-Derivaten. Damit werden Meßfehler bei der Verwendung der neuen Verbindungen in enzymatischen Tests verringert oder sogar beseitigt.

Tabelle IV

Spaltungsgeschwindigkeiten einiger erfindungsgemäßer Thrombinsubstrate in Δ OD/min bei 405 nm im Vergleich zu Substraten nach dem Stand der Technik.

| Synthetische Substrate 3 mMol/1 | Aktivator 2700FE/ml | Thrombin 6IU/ml | Plasmin 2CTA/ml | Kallikrein 0,85BAEE/ml | F XII a 3,6IU/ml |
|---|---|---|---|---|---|
| Substrate für Thrombin nach dem Stand der Technik: | | | | | |
| Tos-Gly-Pro-Arg-pNA | 0,260 | 0,430 | 0,460 | 0,240 | 0,180 |
| H-D-Phe-Pip-Arg-pNA | 0,105 | 0,350 | 0,110 | 0,210 | 0,063 |
| H-D-Phe-Pro-Arg-pNA | 0,115 | 0,350 | 0,230 | 0,220 | 0,068 |
| H-D-CHA-But-Arg-pNA | 0,140 | 0,300 | 0,300 | 0,680 | 0,100 |
| Erfindungsgemäße Substrate für Thrombin: | | | | | |
| H-D-Phe-Pro-Arg-2-methoxy-pNA | 0,090 | 0,340 | 0,130 | 0,075 | 0,160 |
| H-D-Phe-Pro-Arg-ANBS-ethylester | 0,180 | 0,360 | 0,180 | 0,180 | 0,030 |
| H-D-Phe-Pro-Arg-ANBS-isopropylamid | 0,090 | 0,350 | 0,045 | 0,020 | 0,012 |
| H-D-Phe-Pro-Arg-ANBS-Ile-O-$CH_3$ | 0,060 | 0,360 | 0,040 | 0,030 | 0,011 |
| H-D-Phe-Pro-Arg-ANBS-dimethylamino-propylamid | 0,070 | 0,345 | 0,020 | 0,015 | 0,010 |
| H-D-Phe-Pro-Arg-ANBS-Ala-isoproylamid | 0,045 | 0,325 | 0,030 | 0,020 | 0,005 |
| Tos-Gly-Pro-Arg-ANBS-isopropylamid | 0,090 | 0,320 | 0,120 | 0,040 | 0,020 |

Tabelle V

Spaltungsgeschwindigkeiten einiger erfindungsgemäßer Substrate für Kallikrein in $\Delta$ OD/min bei 405 nm im Vergleich zu Substraten nach dem Stand der Technik.

| Synthetische Substrate 3 mMol/l | Aktivator 2700FE/ml | Plasmin 2CTA/ml | Kallikrein 0,85BAEE/ml | F XII A 3,6 U/ml | α-Chymotrypsin 2 mg/ml |
|---|---|---|---|---|---|
| Substrate für Kallikrein nach dem Stand der Technik: | | | | | |
| H-D-Pro-Phe-Arg-pNA | 0,980 | 0,345 | 1,46 | 0,570 | 0,040 |
| Bz-Pro-Phe-Arg-pNA | 0,110 | 0,055 | 0,450 | 0 | 0,010 |
| Erfindungsgemäße Substrate für Kallikrein: | | | | | |
| H-D-Pro-Phe-Arg-2-methoxy-pNA | 0,480 | 0,125 | 1,00 | 0,186 | 0,010 |
| H-D-Pro-Phe-Arg-ANBS-ethylester | 0,380 | 0,145 | 1,20 | 0,200 | 0,030 |
| H-D-Pro-Phe-Arg-ANBS-neopentylamid | 0,010 | 0,020 | 0,35 | 0,040 | 0,010 |

Tabelle VI

Spaltungsgeschwindigkeiten einiger erfindungsgemäßer Substrate für Urokinase in $\Delta$ OD/min bei 405 nm im Vergleich zu Substraten nach dem Stand der Technik.

| Synthetische Substrate 3 mMol/1 | Aktivator 2700FE/ml | Urokinase 5000IU/ml | C1-Esterase 160 E/ml | Trypsin 1,4γ/ml | α-Chymotrypsin 2 mg/ml |
|---|---|---|---|---|---|
| Substrate für Urokinase nach dem Stand der Technik: | | | | | |
| Pyr-Gly-Arg-pNA | 0,115 | 0,175 | 0,080 | 0,255 | 0,230 |
| Erfindungsgemäße Substrate für Urokinase: | | | | | |
| Pyr-Gly-Arg-2-methoxy-pNA | 0,110 | 0,205 | 0,065 | 0,090 | 0,040 |
| Pyr-Gly-Arg-ANBS-isopropylamid | 0,035 | 0,125 | 0,012 | 0,035 | 0,060 |
| Pyr-Gly-Arg-ANBS-benzylamid | 0,040 | 0,125 | 0,010 | 0,025 | 0,055 |
| Pyr-Gly-Arg-ANBS-n-butylester | 0,070 | 0,165 | 0,015 | 0,220 | 0,095 |

Tabelle VII

Spaltungsgeschwindigkeiten einiger erfindungsgemäßer Substrate für Plasmin in $\Delta$ OD/min bei 405 nm im Vergleich zu Substraten nach dem Stand der Technik.

| Synthetische Substrate 3,0 mMol/1 | Plasmin 2CTA/ml | Aktivator 2700FE/ml | Kallikrein 0,85BAEE/ml |
|---|---|---|---|
| Substrate nach dem Stand der Technik für Plasmin: | | | |
| H-D-Val-Leu-Lys-pNA | 0,180 | 0,320 | 0,060 |
| H-D-Phe-Tyr-Arg-pNA | 0,430 | 0,950 | 0,590 |
| H-D-Phe-Tyr-Lys-pNA | 0,210 | 0,350 | 0,100 |
| Erfindungsgemäße Substrate für Plasmin: | | | |
| H-D-Phe-Tyr-Lys-ANBS-isoproylamid | 0,165 | 0,155 | 0,010 |
| H-D-Val-Leu-Lys-ANBS-isopropylamid | 0,270 | 0,100 | 0,005 |

Alle in dieser Tabelle genannten Substrate werden von Thrombin und F XII a nicht gespalten.

Tabelle VIII

Spaltungsgeschwindigkeiten einiger erfindungsgemäßer Substrate für Faktor X a in Δ OD/min bei 405 nm im Vergleich zu Substraten nach dem Stand der Technik.

| Synthetische Substrate 3 mMol/1 | Plasmin 2CTA/ml | F X a 0,2U/ml | Kallikrein 0,85BAEE/ml | Urokinase 5000IU/ml | Trypsin 1,4γ/ml |
|---|---|---|---|---|---|
| Substrate nach Stand der Technik für Faktor X a: | | | | | |
| Bz-Ile-Glu(OMe)-Gly-Arg-pNA | 0,020 | 0,110 | 0,015 | 0,035 | 0,140 |
| Z-D-Leu-Gly-Arg-pNA | 0,210 | 0,250 | 0,148 | 0,178 | 0,093 |
| Erfindungsgemäße Substrate für Faktor X a: | | | | | |
| Z-D-Leu-Gly-Arg-2-methoxy-pNA | 0,162 | 0,410 | 0,067 | 0,108 | 0,048 |
| Z-D-Leu-Gly-Arg-ANBS-methylamid | 0,057 | 0,151 | 0,050 | 0,082 | 0,069 |
| Bz-Ile-Glu(OMe)-Gly-Arg-ANBS-methylamid | 0,001 | 0,085 | 0,001 | 0,080 | 0,040 |

Tabelle IX

Spaltungsgeschwindigkeit einiger erfindungsgemäßer Substrate für C1-Esterase in Δ OD/min bei 405 nm.

| Synthetische Substrate 3 mMol/l | C1-Esterase 160 E/ml |
|---|---|
| Boc-Lys(Z)-Gly-Arg-2-methoxy-pNA | 0,34 |
| H-Lys(Z)-Gly-Arg-2-methoxy-pNA | 0,38 |
| H-Lys(Z)-Gly-Arg-2-butoxy-pNA | 0,74 |
| H-Lys(Z)-Gly-Gly-Arg-2-methoxy-pNA | 0,25 |
| H-D-Val-Leu-Arg-2-methoxy-pNA | 0,54 |
| H-D-Val-Leu-Arg-2-butoxy-pNA | 0,97 |

Herstellung der neuen Verbindungen

Bei der Synthese der neuen Verbindugen, die in Tabelle I aufgeführt sind, wurden die einzelnen Reaktionsschritte alle in ähnlicher Weise durchgeführt. Es wird für jeden Syntheseschritt eine allgemeine Beschreibung gegeben.

1. Synthese der chromogenen ANBS-Ester (R≙ $COOR_1$)

a) 1 Mol des entspreuchenden Alkohols wurde bei −5°C unter Feuchtigkeitsausschluß mit 7,9 ml (0,11 Mol) Thionylchlorid versetzt. In diese Lösung wurden bei −5°C 18,2 g (0,1 Mol) ANBS zugegeben. Nach beendeter Zugabe wurde langsam auf 50°C erwärmt und bei dieser Temperatur 4 h gerührt. Das Lösungsmittel wurde im Vakuum abdestilliert und der ölige Rückstand in Chloroform gelöst. Die organische Phase wurde mit 1 M $KHCO_3$, Wasser, 5% Citronensäure und wieder Wasser gewaschen. Getrocknet wurde über $Na_2SO_4$. Nach Filtration wurde das Lösungsmittel in Vakuum abdestilliert. Das Produkt konnte aus einem geeigneten Lösungsmittel umkristallisiert oder durch Kieselgelchromatographie gereinigt werden.

Die Ausbeuten lagen bei 60 Prozent.

b) Alkohole, deren Reaktivität gegenüber Thionylchlorid gering ist wie aliphatische Alkohole ab 6 Kohlenstoffatomen oder araliphatische Alkohole ab 7 Kohlenstoffatomen sowie Alkohole, die bei den oben beschriebenen Reaktionsbedingungen fest sind wie Phenol oder Naphthol wurden in das Kaliumsalz überführt. 0,1 Mol ANBS und 0,1 Mol HOBt wurden in 100 ml DMF gelöst und eine Lösung von 0,1 Mol DCCI in 100 ml DMF während 30 min zugetropft. Nach 1 h Rühren bei RT wurden 0,1 Mol des entsprechenden Kaliumalkoholats zugegeben und 16 h bei RT gerührt. Die Aufarbeitung wurde zie oben durchgeführt.

Die Ausbeuten logen bei 40 Prozent.

2. Synthese der chromogenen ANBS-amide (R ≙ $CONR_2R_3$, $CONH—(CH_2)_n—N(CH_3)_2$, $CO—Y—OR_1$, $CO—Y—NR_2R_3$)

18,2 g (100 mM) 5-Amino-2-nitrobenzoesäure (ANBS) und 15,3 (100 mM) HOBt × $H_2O$ wurden in 100 ml DMF gelöst und eine Lösung von 20,6 g (100 mM) DCCI in 100 ml DMF während 30 min zugetropft. Nach 1 h Rühren bei RT wurden 0,1 Mol der Aminkomponente zugegeben. Lag die Aminkomponente als ihr Salz vor, so wurde zusätzlich 0,1 Mol NMM zugestzt. Nach 16 h Rühren bei RT wurde vom ausgefallenen DCH abfiltriert und das Lösungsmittel im Vakuum entfernt. Der verbleibende ölige Rückstand wurde in EE gelöst und mehrmals mit 5% (w:v) Citronensäure, Wasser, 1 M $KHCO_3$-Lösung und Wasser gewaschen. Nach Trocknen über $Na_2SO_4$ wurde das Lösungsmittel im Vakuum abdestilliert. Das kristalline Produkt wurde aus einem geeigneten Lösungsmittel umkristallisiert. Die Ausbeuten lagen bei 80 bis 90 Prozent.

3. Synthese der chromogenen 2-Alkoxy-pNA-Derivate (R≙ $OR_1$)

7,7 g (50 mM) 2-Amino-5-nitrophenol wurden in 100 ml Aceton gelöst, mit Stickstoff gespült und 7,0 g (50 mM) $K_2CO_3$ und 50 mM Alkylbromid zugegeben. Es wurde unter Rühren 8 h am Rückfluß gekocht. Nach Abkühlen auf 0°C wurde filtriert und das Lösungsmittel im Vakuum abdestilliert. Der Rückstand wurde in Ether gelöst und mit PE (40/60) kristallisiert.

Die Ausbeuten lagen bei 50 Prozent.

4. Kupplung der chromogenen Komponente

50 mM $N^\alpha$, $N^G$-geschütztes Arginin oder $N^\alpha$,$N^\omega$-geschütztes Lysin oder Ornithin, 50 mM chromogenes Amin gemäß 1, 2 oder 3 und 6,8 g (100 mM) Imidazol wurden in 250 ml Pyridin gelöst und die Lösung auf −20°C abgekühlt. Dann wurden unter Beibehaltung dieser Temperatur 7,5 ml (80 mM) $POCl_3$ zugetropft. Nach beendeter Zugabe ließ man langsam auf RT erwärmen. Das Lösungsmittel wurde im Vakuum entfernt und das resultierende Öl in 1 M $KHCO_3$ aufgenommen. Die wässrige Phase wurde dreimal mit EE extrahiert. Die organische Phase wurde mit 1 m $KHCO_3$-Lösung, Wasser, 5% Citronensäure und wieder Wasser gewaschen und über $Na_2SO_4$ getrocknet. Nach Filtration wurde das Lösungsmittel abdestilliert und die Verbindung mit Ether kristallisiert.

Die Ausbeuten lagen bei 40 bis 60 Prozent.

5. Schutzgruppenabspaltung

a) Abspaltung der Benzyloxycarbonylgruppe (Z—) 10 mM geschütztes, chromogenes Aminosäurederivat gemäß 4. wurden in 20 ml HAc gelöst und 30 ml 33% HBr in HAc unter Feuchtigkeitsausschluß bei RT zugegeben. Nach 40 min Rühren bei RT wurde das Lösungsmittel im Vakuum eingedampft und das Produkt mit Ether kristallisiert. Nach mehrmaligem Waschen mit Ether wird die Substanz im Vakuum über fester KOH 20 h getrocknet.

Die Ausbeuten lagen bei 90 Prozent.

b) Abspaltung der t-Butyloxycarbonylgruppe (Boc-) 10 mM geschütztes, chromogenes Aminosäurederivat gemäß 4. wurden in 50 ml 1,2 n HCl in HAc gelöst und 20 min bei RT gerührt. Das Lösungsmittel wurde im Vakuum entfernt und das Produkt mit Ether kristallisiert. Nach mehrmaligem Waschen mit Ether wird die Substanz im Vakuum über fester KOH 20 h getrocknet.

Die Ausbeuten lagen bei 95 Prozent.

6. Kupplungsreaktion

5 mM geschütztes Di- oder Tripeptidderivat, dessen endständige Carboxylgruppe frei ist, wurden in 20 ml DMF gelöst und bei 0°C mit 5 mM DCCI und 5 mM HOBt versetzt. Nach 30 min Rühren bei 0°C wurde auf RT erwärmt und 5 mM chromogenes Aminosäurederivat gemäß 5a oder 5b sowie 5,5 mM NMM zugegeben. Nach 16 h Rühren bei RT wurde vom DCH abfiltriert und das Lösungsmittel im Vakuum abgedampft. Der gelbe Rückstand wurde in MeOH gelöst und auf einer Sephadex$^R$LH 20-Säule chromatographiert. Die Franktionen der reinen Substanz wurden vereinigt und das Lösungsmittel im Vakuum entfernt. Der erhaltene amorphe Feststoff wurde mehrmals mit Ether gewaschen und im Vakuum über $P_4O_{10}$ getrocknet.
Die Ausbeuten lagen bei 70 Prozent.

7. Schutzgruppenabspaltung

a) Abspaltung der t-Butyloxycarbonylgruppe (Boc) und gegebenenfalls der t-Butylestergruppe in der Seitenkette von Glu und Asp.

2 mM geschütztes, chromogenes Peptidderivat wurden in 30 ml 1,2 n HCl/HAc gelöst und 20 min bei RT gerührt. Das Lösungsmittel wurde im Vakuum entfernt und das Produkt mit Ether kristallisiert. Nach mehrmaligem Waschen mit Ether wurde die Substanz am Hochvakuum über festem KOH getrocknet.

b) Abspaltung der Benzyloxycarbonylgruppe (Z) 2 mM geschütztes, chromogenes Peptidderivat wurden in 10 ml HAc gelöst und 15 ml 33% HBr in HAc unter Feuchtigkeitsausschluß bei RT zugegeben. Nach 1 h Rühren bei RT wurde das Lösungsmittel in Vakuum eingedampft und das Produkt mit Ether kristallisiert. Nach mehrmaligem Waschen mit Ether wurde die Substanz in Wasser gelöst, mit einem Ionenaustauscher in das Hydrochlorid überführt und gefriergetrocknet.

c) Abspaltung aller Schutzgruppen 2 mM des geschützten, chromogenen Peptidderivats werden in 10 ml HF in Anwesenheit von 1,0 ml Anisol gelöst und 1 h bei 0°C gerührt. Das HF wird mit einem trockenen Stickstoffstrom entfernt und die Substanz über festem KOH am Hochvakuum getrocknet. Das Produkt wurde mehrmals mit Ether gewaschen, in Wasser gelöst, mit einem Ionenaustauscher in das Hydrochlorid überführt und gefriergetrocknet.

Beispiele für die Verwendung der neuen Verbindungen für Enzym- und Inhibitor-Bestimmungen:

Beispiel 1

Thrombin — Bestimmung

Substrat: H-D-Phe-Pro-Arg-ANBS-neopentylamid

Testansatz:

20 µl eines 1:51 mit physiologischer NaCl vorverdünnten Plasmas wurden mit 500 µl Aktivierungsreagenz (s. unten) 4 min bei 37°C inkubiert. Anschließend wurden 100 µl Substrat-Lösung (3 mMol/l) zugesetzt und Δ E/min photometrisch bei 405 nm bestimmt.
Als Norm diente eine Bezugskurve mit Standard-Human-Plasma.

Aktivierungsreagenz:

PTT-Reagenz (Reagenz zur Bestimmung der partiellen Thrombin-Zeit) der Behringwerke wurde nach Vorschrift in destilliertem Wasser gelöst und 1:4 mit einer Lösung enthaltend 50 µg/ml RVV (Russelviper venom, Sigma); 50 mMol/l Tris; 75 mMol/l NaCl; 7,5 mMol/l $Ca^{2+}$ und 0,1 g/100 ml Human-Albumin verdünnt.

Beispiel 2

Antithrombin III — Bestimmung

Substrat: H-D-Phe-Pro-Arg-ANBS-Ile-methylester

Testansatz:

50 µl eines 1:51 mit physiologischer NaCl vorverdünnten Plasmas wurden mit 1,0 ml einer Humanalpha-Thrombinlösung (0,3 IU/ml; 100 mMol/l Tris; 100 mMol/l NaCl, pH = 8,2) 5 min bei 37°C vorinkubiert. Anschließend wurden 100 µl Substratlösung (2 mMol/l) zugesetzt und Δ E/min photometrisch bei 405 nm bestimmt.
Als Norm diente eine Bezugskurve mit Standard-Human-Plasma.

Beispiel 3

Plasminogen — Bestimmung

Substrat: H-D-Val-Leu-Lys-ANBS-methylester

Testansatz:

20 µl Plasma wurden mit 1,0 ml Streptokinase-Reagenz (1000 Fibr E/ml; 100 mMol/l $KH_2PO_4$; 100 mMol/l NaCl; pH = 7,2) 10 min bei 37°C inkubiert. Anschließend wurden 100 µl Substrat-Reagenz (3 mMol/l) zugesetzt und Δ E/min photometrisch bei 405 nm bestimmt.
Als Norm diente eine Bezugskurve mit Standard-Human-Plasma.

Beispiel 4

$\alpha_2$-Antiplasmin — Bestimmung

Substrat: H-D-Val-Leu-Lys-ANBS-methylamid

Testansatz:

a) 20 µl Plasma wurden mit 1,0 ml Plasmin (human)-Reagenz (0,1 CTA—U/ml; 100 mMol/l $KH_2PO_4$; 150 mMol/l NaCl; 25% Glycerin, pH = 7,2) 1 min bei 37°C vorinkubiert. Anschließend wurden 100 µl Substrat-Reagenz (3 mMol/l) zugesetzt und Δ E/min photometrisch bei 405 nm bestimmt.

Als Norm diente eine Bezugskurve mit Standard-Human-Plasma.

b) 20 µl Plasma wurden mit 1,0 ml Substrat-Reagenz (0,3 mMol/l; 100 mMol/l $KH_2PO_4$; 150 mMol/l NaCl, pH = 7,2) und 100 µl Plasmin-Reagenz (1 CTA-U/ml; 25—50% Glycerin; 2 mMol/l HCl, pH = 2,5) bei 37°C gemischt. Nach 1 min wurde Δ E/min bestimmt.

Als Norm diente eine Bezugskurve mit Standard-Human-Plasma.

Beispiel 5

Faktor Xa — Bestimmung

Substrat: Z-D-Leu-Gly-Arg-2-methoxy-pNA

Testansatz:

20µl Plasma wurden mit 1 ml Aktivierungsreagenz bei 37°C 1 min vorinkubiert. Anschließend wurden 100 µl Substrat-Reagenz (3 mMol/l) zugesetzt und Δ E/min bestimmt.

Als norm diente eine Bezugskurve mit Standard-Human-Plasma.

Aktivierungsreagenz:

25 µg RVV/ml (Russelviper venom Sigma); 50 mMol/l Tris; 25 mMol/l $CaCl_2$; 200 mMol/l NaCl, pH = 8,3.

Beispiel 6

Prekallikrein — Bestimmung

Substrat: H-D-Pro-Phe-Arg-ANBS-isopropylamid

Testansatz:

10 µl Plasma wurden mit 1 ml Aktivierungsreagenz 1 min bei 37°C inkubiert. Anschließend wurden 100 µl Substrat-Reagenz (3 mMol/l) zugesetzt und Δ E/min bestimmt.

Als Norm diente eine Bezugskurve mit Standard-Human-Plasma.

Aktivierungsreagenz:

Optisch klares PTT-Reagenz der Behringwerke wurde nach Vorschrift in destilliertem Wasser gelöst und 1:6 mit destilliertem Wasser verdünnt. Eine weitere 1:9 Verdünnung wurde mit 50 mMol/l Tris, 12 mMol/l NaCl, pH = 7,8 durchgeführt.

Beispiel 7

Urokinase — Bestimmung

Substrat: Pyr-Gly-Arg-ANBS-benzylamid

Testansatz:

10 µl Urokinase (100—3000 IU/ml) wurden bei 37°C mit 1 ml Triäthanolamin-Puffer (0,1 mol/l TÄA; 0,2 mMol/l NaCl, pH = 8,4) und 1 ml Substrat-Reagenz (2 mMol/l) versetzt und anschließend Δ E/min bestimmt.

Ausgewertet wurde mit Hilfe einer Bezugskurve.

Urokinase — Bestimmung aus Urin

500 µl Urin wurden bei 37°C mit 500 µl Triäthanolamin-Puffer (0,5 mMol/ TÄA; 1 mol/l NaCl, pH = 8,4) und 0,1 ml Substrat-Reagenz (2 mMol/l) versetzt und Δ E/min bestimmt.

Ausgewertet wurde mit Hilfe einer Bezugskurve.

Beispiel 8

C1-Inaktivator — Bestimmung

Substrat: H-D-Val-Leu-Arg-2-butoxy-pNA

Testansatz:

20 µl Plasma wurde mit 1 ml C1-Esterase Reagenz (10 mU/ml; 100 mMol/l $NaH_2PO_4$; 0,05% $NaN_3$; 0,2% Haemaccel; pH = 7,5)15 min bei 30°C inkubiert. Anschließend wurden 100 µl Substrat-Reagenz (6 mMol/l) zugesetzt und Δ E/min photometrisch bei 405 nm bestimmt.

Als Norm diente eine Bezugskurve mit Standard-Human-Plasma.

**Patentansprüche**

1. Chromogene Verbindungen der allgemeinen Formel I

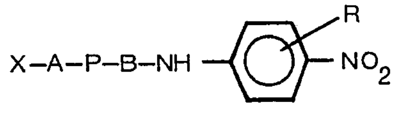

(I)

worin bedeuten

X ein Wasserstoffatom, eine in der Peptidchemie übliche Schutzgruppe oder eine die endständige Aminogruppe irreversibel blockierende Gruppierung,

A und P, gleich oder verschieden, eine aus 2 bis 15 Kohlenstoffatomen mit bis zu 3 Stickstoffatomen, 2 Schwefelatomen und 6 Sauerstoffatomen bestehende alpha-, beta- oder gamma-Aminosäure und P außerdem ein Dipeptid, gebildet aus solchen Aminosäuren,

B Arginin, Homoarginin, Lysin, Homolysin, Ornithin oder Histidin und

R ein Substituent in 2- oder 3-Stellung, der eine Ether-, Ester-, Amid-, Thioester- oder araliphatische Bindung enthält, über die er mit dem aromatischen Kern verbunden sein kann, und aus 1 bis 30 Kohlenstoffatomen und der dazugehörigen Anzahl von Wasserstoffatomen besteht, wobei 1 bis 10 Kohlenstoffatome durch Sauerstoff, Stickstoff oder Schwefel und 2 bis 10 Wasserstoffatome durch Gruppen, die Sauerstoff oder Schwefel enthalten, ersetzt sein können,

sowie ihre Säureadditionssalze.

2. Verbindung nach Anspruch 1, dadurch gekennzeichnet, daß A eine chirale Aminosäure ist.

3. Verbindung nach Anspruch 2, dadurch gekennzeichnet, daß A eine chirale Aminosäure in der D-Form ist, wenn X ein Wasserstoffatom ist.

4. Verbindung nach Anspruch 1, gekennzeichnet durch die allgemeine Formel II

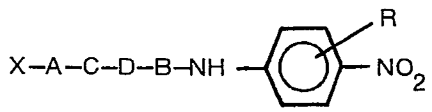

(II)

worin

X ein Wasserstoffatom, eine in der Peptidchemie übliche Schutzgruppe oder eine die endständige Aminogruppe irreversibel blockierende Gruppierung,

A eine Aminosäure, ausgewählt aus der Gruppe Ala, Cys, Glu, Gln, Gly, His, Ile, Leu, Lys, Phe, Pro, Pyr, Thr, Tyr und Val,

C eine Bindung oder eine Aminosäure, ausgewählt aus der Gruppe Ala, Asp, Glu, Gly, Leu, Lys, Ser und Val,

D eine Aminosäure, ausgewählt aus der Gruppe Ala, Asp, Glu, Gly, His, Leu, Phe, Pip, Pro, Ser, Thr, Tyr und Val,

B L-Arginin, L-Homoarginin, L-Lysin, L-Homolysin, L-Ornithin oder L-Histidin und

R $COOR_1$, $CONR_2R_3$, $CONH—(CH_2)_n—N(CH_3)_2$, $CO—Y—OR_1$ und $CO—Y—NR_2R_3$ in 3-Stellung oder $OR_1$ in 2-Stellung des 4-Nitroanilins bedeutet, wobei

$R_1$ ein aliphatischer Kohlenwasserstoffrest mit 1 bis 6 Kohlenstoffatomen, ein aromatischer Kohlenwasserstoffrest mit 6 bis 10 Kohlenwasserstoffatomen oder ein araliphatischer Kohlenwasserstoffrest mit 7 bis 11 Kohlenstoffatomen oder ein alicyclischer Kohlenwasserstoffrest mit 3 bis 8 Kohlenstoffatomen,

$R_2$ ein Wasserstoffatom oder ein unter $R_1$ definierter Rest,

$R_3$ ein aliphatischer Kohlenwasserstoffrest mit 1 bis 10 Kohlenstoffatomen, ein aromatischer Kohlenwasserstoffrest mit 6 oder 10 Kohlenstoffatomen, ein araliphatischer Kohlenwasserstoffrest mit 7 bis 11 Kohlenstoffatomen oder ein alicyclischer Kohlenwasserstoffrest mit 3 bis 8 Kohlenstoffatomen,

Y eine alpha-, beta- oder gamma-Aminosäure ausgewählt aus der Gruppe Ala, Asn, Asp, β-Ala, gamma-But, Cys, Glu, Gly, Ile, Leu, Lys, Met, Arg, Phe, Pro, Ser, Thr, Tyr und Val und

n 1 bis 10 bedeuten,

und ihre Säureadditionssalze.

5. Verbindung nach Anspruch 1, gekennzeichnet durch die allgemeine Formel II

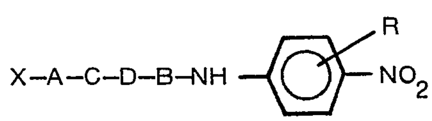

(II)

worin

X ein Wasserstoffatom, Benzyloxycarbonyl, tert.-Butyloxycarbonyl, Adamantyloxycarbonyl, Methylsulfonethyloxycarbonyl oder 9-Fluorenylmethyloxycarbonyl oder eine $R_4—CO$-Gruppe, worin $R_4$ einen aliphatischen Kohlenwasserstoffrest mit 1 bis 6 Kohlenstoffatomen, der mit 1 bis 3 Halogenatomen substituiert sein kann, oder eine Alkaryl-Gruppe mit 6 bis 10 Kohlenstoffatomen oder die Benzolsulfonyl- oder eine Alkarylsulfonyl-Gruppe mit 7 bis 10 Kohlenstoffatomen bedeutet, und A, C, D, B und R wie in Anspruch 4 definiert sind,

und ihre Säureadditionssalze.

6. Verbindung nach Anspruch 1, gekennzeichnet durch die allgemeine Formel II

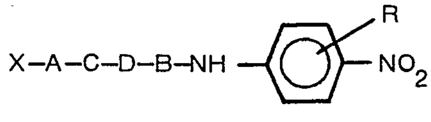

(II)

worin

X Formyl-, Acetyl-, Benzoyl-, Trifluoracetyl-, Toluolsulfonyl-, Mesyl-, Methoxybenzolsulfonyl-, Succinoyl- oder Maleoyl- ist und A, C, D, B und R wie in Anspruch 4 definiert sind,

und ihre Säureadditionssalze.

7. Verfahren zur Herstellung einer Verbindung der Formel I, dadurch gekennzeichnet, daß ein Peptidderivat der allgemeinen Formel III

$$X—A—P—OH \qquad (III)$$

worin X, A und P die oben angegebene Bedeutung haben, wobei jedoch X kein Wasserstoffatom bedeutet und zusätzliche funktionelle Gruppen in den Aminosäureseitenketten mit Schutzgruppen substituiert sind, mit einem Aminosäurederivat der allgemeinen Formel IV kondensiert wird,

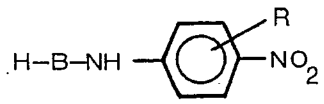

(IV)

worin B die oben genannte Bedeutung besitzt, wobei jedoch zusätzliche funktionelle Gruppen in der Seitenkette der Aminosäure durch Schutzgruppen substituiert sind, und R die zu der allgemeinen Formel I gegebene Definition hat und vorhandene Schutzgruppen gegebenenfalls abgespalten werden.

8. Verwendung einer Verbindung nach einem der Ansprüche 1 bis 6 zum Nachweis und zur Bestimmung eines Enzyms.

**Revendications**

1. Composés chromogènes de formule générale I

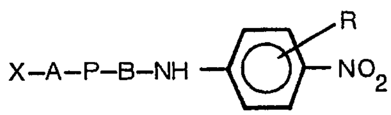

(I)

dans laquelle

X représente un atome d'hydrogène, un groupe protecteur usuel dans la chimie des peptides ou un groupement bloquant irréversiblement le groupe amino terminal;

A et P, identiques ou différents, représentent un α, β ou γ-aminoacide constitué de 2 à 15 atomes de carbone avec jusqu'à 3 atomes d'azote, 2 atomes de soufre et 6 atomes d'oxygène, et P représente en outre un dipeptide formé à partir de tels aminoacides;

B représente l'arginine, l'homoarginine, la lysine, l'homolysine, l'ornithine ou l'histidine;

R représente un substituant en position 2 ou 3, qui contient une liaison éther, ester, amide, thioester ou araliphatique par laquelle il peut être lié au noyau aromatique, et est constitué de 1 à 3 atomes de carbone et du nombre correspondant d'atomes d'hydrogène, de 1 à 10 atomes de carbone pouvant être remplacés par des atomes d'oxygène, azote ou soufre, et de 2 à 10 atomes d'hydrogène pouvant être remplacés par des groupes qui contiennent de l'azote ou du soufre,

et sels d'addition avec des acides de ceux-ci.

2. Composé selon la revendication 1, caractérisé en ce que A est un aminoacide chiral.

3. Composé selon la revendication 1, caractérisé en ce que A est un aminoacide chiral sous la forme D, lorsque X est un atome d'hydrogène.

4. Composé selon la revendication 1, caractérisé par la formule générale II

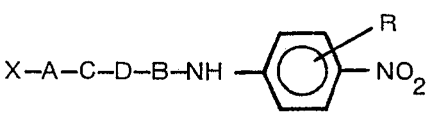

(II)

dans laquelle

X représente un atome d'hydrogène, un groupe protecteur usuel dans la chimie des peptides ou un groupement bloquant irréversiblement le groupe amino terminal;

A représente un aminoacide choisi parmi Ala, Cys, Glu, Gln, Gly, His, Ile, Leu, Lys, Phe, Pro, Pyr, Thr, Tyr et Val;

C représent une liaison ou un aminoacide choisi parmi Ala, Asp, Glu, Gly, Leu, Lys, Ser et Val;

D représente un aminoacide choisi parmi Ala, Asp, Glu, Gly, His, Leu, Phe, Pip, Pro, Ser, Thr, Tyr et Val;

B représente la L-arginine, la L-homoarginine, la L-lysine, la L-homolysine, la L-ornithine ou la L-histidine; et

R représente $COOR_1$, $CONR_2R_3$, $CONH—(CH_2)_n—N(CH_3)_2$, $CO—Y—OR_1$ et $CO—Y—NR_2R_3$ en position 3, ou $OR_1$ en position 2 de la 4-nitroaniline,

$R_1$ étant un radical hydrocarboné aliphatique ayant de 1 à 6 atomes de carbone, un radical hydrocarboné aromatique ayant de 6 à 10 atomes de carbone ou un radical hydrocarboné araliphatique ayant de 7 à 11 atomes de carbone, ou un radical hydrocarboné alicyclique ayant de 3 à 8 atomes de carbone;

$R_2$ étant un atome d'hydrogène ou un radical tel que défini en $R_1$;

$R_3$ représentant un radical hydrocarboné aliphatique ayant de 1 à 10 atomes de carbone, un radical hydrocarboné aromatique ayant 6 ou 10 atomes de carbone, un radical hydrocarboné araliphatique ayant de 7 à 11 atomes de carbone ou un radical hydrocarboné alicyclique ayant de 3 à 8 atomes de carbone;

Y représentant un α-, β- ou γ-aminoacide choisi parmi Ala, Asn, Asp, β-Ala, γ-But, Cys, Glu, Gly, Ile, Leu, Lys, Met, Arg, Phe, Pro, Ser, Thr et Tyr, Val; et

n allant de 1 à 10,

et sels d'addition avec des acides de celui-ci.

5. Composé selon la revendication 1, caractérisé par la formule générale II

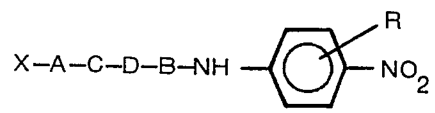

(II)

dans laquelle

X représente un atome d'hydrogène, le groupe benzyloxycarbonyle, tert-butyloxycarbonyle, adamantyloxycarbonyle, méthylsulfonyléthyloxycarbonyle ou 9-fluorénylméthyloxycarbonyle ou un groupe $R_4CO$ dans lequel $R_4$ représente un radical hydrocarbone aliphatique ayant de 1 à 6 atomes de carbone, qui peut être substitué par 1 à 3 atomes d'halogène, ou un groupe aralkyle ayant de 6 à 10 atomes de carbone ou le groupe benzènesulfonyle ou un groupe alkarylsulfonyle ayant de 7 à 10 atomes de carbone, et

A, C, D, B et R sont tels que définis dans la revendication 4,

et sels d'addition avec des acides de celui-ci.

6. Composé selon la revendication 1, caractérisé par la formule générale II

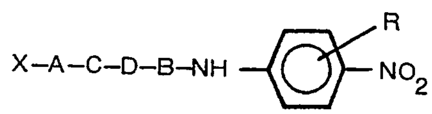

(II)

dans laquelle

X est le groupe formyle, acétyle, benzoyle, trifluoroacétyle, toluènesulfonyle, mésyle, méthoxybenzènesulfonyle, succinoyle ou maléoyle, et

A, C, D, B et R sont tels que définis dans la revendication 4,

et sels d'addition avec des acides de celui-ci.

7. Procédé pour la préparation d'un composé de formule I, caractérisé en ce que l'on condense un dérivé de peptide de formule générale III

$$X—A—P—OH \quad (III)$$

dans laquelle X, A et P ont les significations données plus haut, X ne représentant toutefois pas un atome d'hydrogène, et des groupes fonctionnels supplémentaires sur les chaînes latérales d'aminoacides étant substitués par des groupes protecteurs, avec un dérivé d'aminoacide de formule générale IV

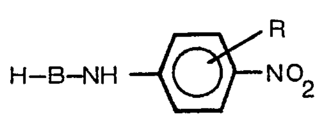

(IV)

dans laquelle B a la signification donnée plus haut, des groupes fonctionnels supplémentaires sur la chaîne latérale de l'aminoacide étant toutefois substitués par des groupes protecteurs, et R a la définition donnée à propos de la formule générale I, et on élimine éventuellement des groupes protecteurs présents.

8. Utilisation d'un composé selon l'une des revendications 1 à 6, pour la mise en évidence et pour le dosage d'une enzyme.

## Claims

1. A chromogenic compound of the formula I

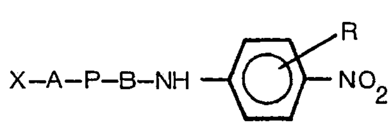

(I)

in which

X denotes a hydrogen atom, a protective group customarily used in peptide chemistry or a group which irreversibly blocks the terminal amino group,

A and P are identical or different and denote an alpha-, beta- or gamma-amino acid which is composed of 2 to 15 carbon atoms with up to 3 nitrogen atoms, 2 sulfur atoms and 6 oxygen atoms, and P also denotes a dipeptide formed from amino acids of these types,

B denotes arginine, homoarginine, lysine, homolysine, ornithine or histidine and

R denotes a substituent in the 2- or 3-position, which contains an ether, ester, amide, thioester or araliphatic bond via which it can be bonded to the aromatic nucleus, and is composed of 1 to 30 carbon atoms and the relevant number of hydrogen atoms, it being possible for 1 to 10 carbon atoms to be replaced by oxygen, nitrogen or sulfur, and for 2 to 10 hydrogen atoms to be replaced by groups which contain oxygen or sulfur,

and its acid addition salts.

2. A compound as claimed in claim 1, wherein A is a chiral amino acid.

3. A compound as claimed in claim 2, wherein A is a chiral amino acid in the D form when X is a hydrogen atom.

4. A compound as claimed in claim 1, which has the formula II

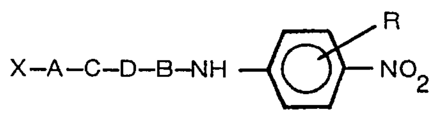

(II)

in which

X denotes a hydrogen atom, a protective group customarily used in peptide chemistry or a group which irreversibly blocks the terminal amino group,

A denotes an amino acid, selected from the group comprising Ala, Cys, Glu, Gln, Gly, His, Ile, Leu, Lys, Phe, Pro, Pyr, Thr, Tyr and Val;

C denotes a bond or an amino acid, selected from the group comprising Ala, Asp, Glu, Gly, Leu, Lys, Ser and Val;

D denotes an amino acid, selected from the group comprising Ala, Asp, Glu, Gly, His, Leu, Phe, Pip, Pro, Ser, Thr, Tyr and Val,

B denotes L-arginine, L-homoarginine, L-lysine, L-homolysine, L-ornithine or L-histidine, and

R denotes $COOR_1$, $CONR_2R_3$, $CONH—(CH_2)_n—N(CH_3)_2$, $CO—Y—OR_1$ and $CO—Y—NR_2R_3$ in the 3-position or $OR_1$ in the 2-position of the 4-nitroaniline,

in which

$R_1$ denotes an aliphatic hydrocarbon radical having 1 to 6 carbon atoms, an aromatic hydrocarbon radical having 6 to 10 carbon atoms or an araliphatic hydrocarbon radical having 7 to 11 carbon atoms or an alicyclic hydrocarbon radical having 3 to 8 carbon atoms,

$R_2$ denotes a hydrogen atom or a radical defined under $R_1$,

$R_3$ denotes an aliphatic hydrocarbon radical having 1 to 10 carbon atoms, an aromatic hydrocarbon radical having 6 or 10 carbon atoms, an araliphatic hydrocarbon radical having 7 to 11 carbon atoms or an alicyclic hydrocarbon radical having 3 to 8 carbon atoms,

Y denotes an alpha-, beta- or gamma-amino acid, selected from the group comprising Ala, Asn, Asp, β-Ala, gamma-But, Cys, Glu, Gly, Ile, Leu, Lys, Met, Arg, Phe, Pro, Ser, Thr, Tyr and Val and

n denotes 1 to 10,

and its acid addition salts.

5. A compound as claimed in claim 1, which has the formula II

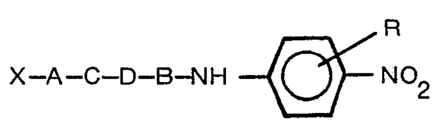

(II)

in which X denotes a hydrogen atom, benzyloxycarbonyl, tert.-butyloxycarbonyl, adamantyloxycarbonyl, methylsulfoethyloxycarbonyl or 9-fluoroenylmethyloxycarbonyl or an $R_4$—CO group, in which $R_4$ denotes an aliphatic hydrocarbon radical having 1 to 6 carbon atoms, which can be substituted with 1 to 3 halogen atoms, or an alkaryl group having 6 to 10 carbon atoms, or the benzenesulfonyl or an alkarylsulfonyl group having 7 to 10 carbon atoms, and A, C, D, B and R are defined as in Claim 4, and its acid addition salts.

6. A compound as claimed in claim 1, which has the formula II

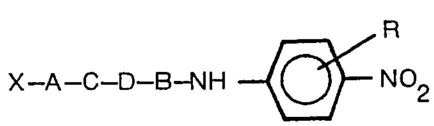

(II)

in which X is formyl acetyl, benzoyl, trifluoroacetyl, toluenesulfonyl, mesyl, methoxybenzenesulfonyl, succinoyl or maleoyl, and A, C, D, B and R are defined as in claim 4, and its acid addition salts.

7. A process for the preparation of a compound of the formula I, which comprises condensing a peptide derivative of the formula III

$$X—A—P—OH \qquad (III)$$

in which X, A and P have the abovementioned meaning, X not, however, denoting a hydrogen atom, and additional functional groups in the amino acid side-chains being substituted with protective groups, with an amino acid derivative of the formula IV

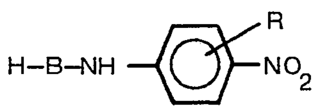

(IV)

in which B has the abovementioned meaning, but additional functional groups in the side-chain of the amino acid being substituted by protective groups, and R having the definition given for the formula I, and splitting off, where appropriate, protective groups which are present.

8. The use of a compound as claimed in one of claims 1 to 6 for the detection and for the determination of an enzyme.